(19) Europäisches Patentamt
European Patent Office
Office européen des brevets

(11) EP 4 321 508 B1

(12) **EUROPEAN PATENT SPECIFICATION**

(45) Date of publication and mention of the grant of the patent:
**02.09.2026 Bulletin 2026/36**

(21) Application number: **22810633.2**

(22) Date of filing: **26.05.2022**

(51) International Patent Classification (IPC):
***C07D 307/93*** $^{(2006.01)}$ ***C07D 405/06*** $^{(2006.01)}$
***A61K 31/365*** $^{(2006.01)}$ ***A61K 31/5377*** $^{(2006.01)}$
***A61K 31/495*** $^{(2006.01)}$ ***A61K 31/496*** $^{(2006.01)}$
***A61P 29/00*** $^{(2006.01)}$ ***C07D 307/94*** $^{(2006.01)}$

(52) Cooperative Patent Classification (CPC):
**A61P 29/00; C07D 307/93; C07D 307/94; C07D 405/06;** A61K 31/365; A61K 31/496; A61K 31/5377

(86) International application number:
**PCT/CN2022/095243**

(87) International publication number:
**WO 2022/247909 (01.12.2022 Gazette 2022/48)**

(54) **GUAIANE SESQUITERPENE DERIVATIVES AND PHARMACEUTICAL USE THEREOF**

GUAIAN-SESQUITERPEN-DERIVATE UND IHRE PHARMAZEUTISCHE VERWENDUNG

DÉRIVÉS DE SESQUITERPÈNE DE GUAIANE ET LEUR UTILISATION PHARMACEUTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.05.2021 CN 202110588960**
**25.05.2022 CN 202210573181**

(43) Date of publication of application:
**14.02.2024 Bulletin 2024/07**

(73) Proprietor: **Nanjing University Of Chinese Medicine**
**Nanjing, Jiangsu 210023 (CN)**

(72) Inventors:
- **HU, Lihong**
**Nanjing, Jiangsu 210023 (CN)**
- **LIU, Jian**
**Nanjing, Jiangsu 210023 (CN)**
- **LV, Qi**
**Nanjing, Jiangsu 210023 (CN)**
- **HU, Yang**
**Nanjing, Jiangsu 210023 (CN)**
- **DONG, Dong**
**Nanjing, Jiangsu 210023 (CN)**
- **WANG, Ping**
**Nanjing, Jiangsu 210023 (CN)**
- **YANG, Meng**
**Nanjing, Jiangsu 210023 (CN)**

(74) Representative: **Murgitroyd & Company**
**165-169 Scotland Street**
**Glasgow G5 8PL (GB)**

(56) References cited:
**CN-A- 102 731 454** **CN-A- 103 417 532**
**CN-A- 103 622 954** **US-A1- 2020 277 270**

- **ABDERRAZAK A. ET AL: "Inhibition of the Inflammasome NLRP3 by Arglabin Attenuates Inflammation, Protects Pancreatic -Cells from Apoptosis, and Prevents Type 2 Diabetes Mellitus Development in ApoE2Ki Mice on a Chronic High-Fat Diet", vol. 357, no. 3, 4 April 2016 (2016-04-04), US, pages 487 - 494, XP093193769, ISSN: 1521-0103, Retrieved from the Internet <URL:https://jpet.aspetjournals.org/content/jpet/357/3/487.full.pdf> DOI: 10.1124/jpet.116.232934**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 321 508 B1

- DING YA-HUI; GAO XUE; LONG JING; KUANG BEI-JIA; CHEN YUE; ZHANG QUAN: "Dispirocyclopropyldehydrocostus lactone selectively inhibits acute myelogenous leukemia cells", BIOORGANIC & MEDICINAL CHEMISTRY LETTERS, vol. 26, no. 4, 18 January 2016 (2016-01-18), Amsterdam NL , pages 1165 - 1168, XP029409782, ISSN: 0960-894X, DOI: 10.1016/j.bmcl.2016.01.046
- SALAZAR, I. ; DIAZ, E.: "Carbofluorination of pseudoguaianolide sesquiterpenic lactones", TETRAHEDRON, ELSEVIER SIENCE PUBLISHERS,, vol. 35, no. 7, 1 January 1979 (1979-01-01), AMSTERDAM, NL , pages 815 - 818, XP026658825, ISSN: 0040-4020, DOI: 10.1016/0040-4020(79)80099-X

## Description

### TECHNICAL FIELD

The present invention relates to a sesquiterpene lacton derivatives and use thereof, specifically to a class of guaianolide sesquiterpene lactone derivatives and pharmaceutical use thereof.

### BACKGROUND OF THE INVENTION

Inflammasome is a protein complex in innate immune cells such as macrophages, monocytes and dendritic cells, which can recognize PAMPs (pathogen-associated molecular patterns) or/and DAMPs (damage-associated molecular patterns) [Front Immunol, 2019, 10: 2538.]. Different types of inflammasomes, such as NLRP1, NLRP3, NLRC4, Pyrin, NLRP6 and AIM2, etc., can mediate inflammatory responses, promote the release of inflammatory cytokines, and transmit signals to the immune system, which are the initiators of inflammation and the bridges between natural immunity and acquired immunity [Cell, 2016, 165:792-800.]. Different from other types of inflammasomes that recognize specific DAMPs or PAMPs, the NLRP3 inflammasome can broadly recognize DAMPs and PAMPs from different sources. Therefore, the study of the NLRP3 inflammasome is of great interests in different fields. It is also the most deeply studied inflammasome at present, and has been proved to be involved in the occurrence and development of a variety of chronic inflammatory diseases [Nat Rev Drug Discov, 2018, 17:588-606.].

The NLRP3 flammasome consists of the receptor protein NLRP3, the regulatory protein ASC and the effector protein pro-Caspase-1 [Immunol Rev, 2015, 265:35-52.]. The activation of the NLRP3 inflammasome is divided into two steps: step 1: the TLR4 receptor recognizes the first signal, such as PAMPs, DAMPs or exogenous stress molecules, etc., and up-regulates the protein expression of NLRP3, pro-IL-1β and pro-IL-18 by activating the NF-κB pathway; and step 2: the receptor protein NLRP3 recognizes the second signal, such as PAMPs, DAMPs or intracellular stress molecules, etc., activates pro-Caspase-1 by combining with the adaptor protein ASC, and then cleaves and activates pro-IL-1β and pro-IL-18 to promote the maturation and secretion of IL-1β and IL-18 [Int J Mol Sci, 2019, 20:3328.]. IL-1β will further activate the NF-κB signaling pathway through the autocrine and paracrine pathways, promote the secretion of cytokines such as IL-1β, TNF-α, IL-6 and IL-8, etc., and trigger inflammatory cascade reactions, leading to the occurrence and development of chronic diseases [Front Immunol, 2019, 10:276.].

The overactivation of the NLRP3 inflammasome is closely related to the occurrence and development of many diseases, including an immune disease, an autoimmune disease, a malignant tumor, a skin disease, a cardiovascular disease, a liver-associated disease, a kidney system-associated disease, a gastrointestinal tract-associated disease, a central nervous system disease, a metabolic disease, an endocrine-associated disease, a respiratory disease, a lymphatic system disease, inflammation, an infectious disease, an ocular disease, a psychological disorder and pain, etc. [Nat Med, 2015, 21:248-255; J Clin Invest, 2020, 130:1961-1976; Cell Metab, 2020, 31:580-591; Circ Res, 2018, 122:1722-1740; J Hepatol, 2017, 66:1037-1046; Ageing Res Rev, 2020, 64:101192; Autophagy, 2019, 15:1860-1881; Brain, 2020, 143:1414-1430; Mucosal Immunol, 2019, 12:1150-1163; J Clin Invest, 2018, 128:1793-1806; Immunology, 2020, 160:78-89; J Inflamm (Lond), 2015, 12:41; Nat Commun, 2020, 11:4243; Front Immunol, 2020, 11:570251; BiochemBiophys Res Commun, 2016, 477:329-335; Pharmaceutics, 2020, 12:867; Arthritis Rheumatol, 2020, 72:1192-1202; Food Chem Toxicol, 2020, 144:111588; EMBO Rep, 2020, 21:e49666; Int Immunopharmacol, 2020, 81:106257; Cells, 2019, 8: 1389; Cell Prolif, 2021, 54: e12973.]. Therefore, the above disease can be prevented and/or treated by inhibiting the activation of the NLRP3 inflammasome.

The structural formula of arglabin is as follows:

O H O O .

Abderrazak A's group found that arglabin, a guaianolide sesquiterpene lactone, exhibited extremely potent inhibitory activity against the NLRP3 inflammasome activation ($EC_{50}$ = 10 nM). Arglabin can alleviate the NLRP3-related inflammatory diseases, protect pancreatic β-cells and prevent type 2 diabetes [Circulation, 2015, 131:1061-1070; J Pharmacol Exp Ther, 2016, 357:487-494.]. Arglabin is derived from the plant Wormwood (Artemisia) in Kazakhstan, with a low content of about 0.27% [J Nat Prop, 1999, 62: 1068-1071.]; its water-solubility was only 7.9 μg/mL; and it has a poor

chemical stability in gastric juice environments, with the degradation ratio of 50% within 8 h, and the oral bioavailability of only 5%, which shortcomings in druggability limit its clinical application. Therefore, the chemical stability, water solubility, oral bioavailability and resource supply economy of such compounds need to be further improved.

## SUMMARY OF THE INVENTION

The present invention does not relate to methods for treatment of the human or animal body by surgery or therapy or to diagnostic methods practiced on the human or animal body. Any references to such methods within this document are to be understood as not forming part of the claimed invention, and are included solely for illustrative purposes or to provide context for the technical aspects of the invention.

Purpose of the present invention: The purpose of the present invention is to provide a guaianolide sesquiterpene lactone derivative to improve the chemical stability, water solubility and oral bioavailability of such compounds. Another purpose of the present invention is to provide use of such compounds in preparation of a medicine for treating an NLRP3 inflammasome-associated disease.

Technical scheme: Guaianolide sesquiterpene lactone derivatives of general formula I, or a pharmaceutically acceptable salt thereof, wherein

I

$R_1$ and $R_2$ together form a double bond; or $R_1$ is hydrogen or deuterium, $R_2$ is

$$-CH_2N(R_3)(R_4),$$

wherein $R_3$ and $R_4$ are hydrogen, alkyl or cycloalkyl respectively; and $R_3$, $R_4$ and a N atom form a 3~9 membered ring structure which can be substituted by one or more substituents, including an alkyl, ester group, aryl, alkylaryl, arylalkyl, arylalkenyl, arylalkynyl or heterocyclyl;

$R_5$ can be connected with $R_6$ by a single bond to form cyclopropane, and when $R_5$ and $R_6$ do not form cyclopropane, $R_5$ is methyl, and $R_6$ is a hydroxyl, alkoxy, ester group or halogen, etc., and when $R_6$ is not a hydroxyl, alkoxy, ester group or halogen, it can form a double bond with an adjacent carbon atom, respectively;

$R_7$ is hydrogen or hydroxyl; and

$R_8$ can be connected with $R_9$ by a single bond to form cyclopropane, and $R_{10}$ is hydrogen; and when $R_8$ and $R_9$ do not form cyclopropane, $R_8$ is methyl, and $R_9$ is connected with $R_{10}$ directly to form cyclopropane.

Further preferably, the guaianolide sesquiterpene lactone derivative or a pharmaceutically acceptable salt thereof is selected from the following compounds:

1 2 3 4

5 6 7

9

10 11 12 13

14 15 16

R= 18 19 20 21 22 23

Preferably, the guaianolide sesquiterpene lactone derivatives or a pharmaceutically acceptable salt thereof refers to a pharmaceutically acceptable salt formed with an inorganic or organic acid, including a hydrochloride, a sulfate, a phosphate, a maleate, a fumarate, and a citrate, etc.

Further, the pharmaceutically acceptable salt is selected from:

24, 25, 26, 27,

28 , 29 , 30 , 31

This application further discloses a method for preparation of the guaianolide sesquiterpene lactone derivatives above.

This application further discloses a pharmaceutical composition comprising a therapeutically effective amount of one or more selected from the guaianolide sesquiterpene lactone derivatives above or a pharmaceutically acceptable salt thereof as active ingredients. The pharmaceutical composition further comprises a pharmaceutically acceptable carrier, adjuvant or auxiliary material.

This application further discloses use of the guaianolide sesquiterpene lactone derivatives or a pharmaceutically acceptable salt thereof and the pharmaceutical composition above in preparation of a medicine for preventing or treating NLRP3 inflammasome-associated diseases.

This application further discloses use of the guaianolide sesquiterpene lactone derivatives above or a pharmaceutically acceptable salt thereof in combination with other pharmaceutically acceptable therapeutic agents, especially other NLRP3 inflammasome inhibitors, in preparation of a medicine for preventing or treating NLRP3 inflammasome-associated diseases.

The present invention provides a method for preventing or treating NLRP3 inflammasome-associated diseases, including administering a therapeutically effective amount of one or more selected from the guaianolide sesquiterpene lactone derivatives according to the present invention or a pharmaceutically acceptable salt thereof, or the pharmaceutical composition according to the present invention comprising a therapeutically effective amount of one or more selected from the guaianolide sesquiterpene lactone derivatives according to the present invention or a pharmaceutically acceptable salt thereof as active ingredients to a patient in need thereof.

The NLRP3 inflammasome-associated diseases includes: immune diseases, autoimmune diseases, malignant tumor, skin diseases, cardiovascular diseases, liver-associated diseases, kidney system-associated diseases, gastrointestinal tract-associated diseases, central nervous system diseases, metabolic diseases, endocrine-associated diseases, respiratory diseases, lymphatic system diseases, inflammation, infectious diseases, ocular disease, psychological disorder and pain, etc.

Specifically, the disease includes: (1) Cryopyrin-associated periodic syndrome (CAPS): a Muckle-Wells syndrome (MWS), a familial cold autoinflammatory syndrome (FCAS) and a neonatal-onset multisystem inflammatory disease (NOMID); (2) autoinflammatory diseases: familial Mediterranean fever (FMF), a TNF receptor-associated periodic syndrome (TRAPS), mevalonate kinase deficiency (MKD), a hyperimmunoglobulin D and periodic fever syndrome (HIDS), deficiency of interleukin-1 receptor (DIRA), a Majeed syndrome, pyogenic arthritis, pyoderma gangrenosum and acne (PAPA), haploinsufficiency of A20 (HA20), pediatric granulomatous arthritis (PGA), PLCG2-associated antibody deficiency and immune dysregulation (PLAID), PLCG2-associated autoinflammation, antibody deficiency and immune dysregulation (APLAID), and sideroblastic anaemia with B-cell immunodeficiency, periodic fever and developmental delay (SIFD); (3) Sweet's syndrome: chronic nonbacterial osteomyelitis (CNO), chronic recurrent multifocal osteomyelitis (CRMO), and a synovitis, acne, pustulosis, hyperostosis, and osteitis syndrome (SAPHO); (4) autoimmune diseases: multiple sclerosis (MS), type 1 diabetes, psoriasis, rheumatoid arthritis, a Behcet's disease, a Sjogren's syndrome, and a Schnitzler syndrome; (5) respiratory system diseases: a chronic obstructive pulmonary disorder (COPD), steroid-resistant asthma, asbestosis, silicosis and cystic fibrosis; (6) central nervous system diseases: a Parkinson's disease, an Alzheimer's disease, a motor neuron disease, a Huntington's disease, cerebral malaria and brain injury from pneumococcal meningitis; (7) metabolic diseases: type 2 diabetes, atherosclerosis, obesity, gout and pseudogout; (8) ocular diseases: an ocular epithelium disease, age-related macular degeneration (AMD), corneal infection, uveitis and xerophthalmia; (9) kidney-associated diseases: a chronic kidney disease, oxalate nephropathy and diabetic nephropathy; (10) liver-associated diseases: non-alcoholic steatohepatitis and an alcoholic liver disease; (11) skin-associated inflammatory reaction: contact hypersensitivity and sunburn; (12) joint-associated inflammatory reaction: osteoarthrosis, systemic juvenile idiopathic arthritis, an adult Still's disease, and relapsing polychondritis; (13) viral infection: Dengue virus and Zika virus, influenza, and HIV virus; (14) hidradenitis suppurativa (HS) and other cyst-causing skin diseases; (15) cancer: lung cancer, pancreatic cancer, gastric cancer, a myelodysplastic syndrome, abdominal aortic aneurysm and leukemia; and (16) polymyositis, colitis, pericarditis, worm infection, bacterial infection, wound healing, depression, stroke, myocardial infarction, hypertension, a Dressler's syndrome, and ischemia-reperfusion injury disease, etc.

The colitis includes ulcerative colitis.

The NLRP3 inflammasome-associated disease includes acute gouty arthritis.

Beneficial effect: Compared with the existing technology, in the present application, the guaianolide sesquiterpene lactone derivatives of general formula I are synthesized with high stereoselectivity by specific structural modification with abundant natural ingredients such as parthenolide and dehydrocostus lactone as raw materials, and the cyclopropane configurations of the obtained compounds are all $\alpha$ configurations. The experimental results show that the inhibitory activity of these compounds on the activation of the NLRP3 inflammasome is maintained, and their chemical stability, water solubility and oral bioavailability are significantly improved, so they have better development and application prospects.

## BRIEF DESCRIPTION OF THE DRAWING

Fig. 1 shows the change of the concentrations of Compound 1 and Compound 25 in the HEPES7.4 solution with time; and

Fig. 2 shows the change of the concentrations of Compound 1 and Compound 25 in mouse plasma with time.

## DETAILED DESCRIPTION

This application will be described in detail in combination with specific examples.

Example compounds 8, 17 and 32 do not form part of the invention.

### Example 1: Preparation of parthenolide and dehydrocostus lactone

Preparation of parthenolide. 5 kg of the dried root bark of *Magnolia delavayi* was crushed into coarse powder, soaked in 10× 95% ethanol for 12 h, heated, refluxed and extracted twice with 2 h each time, and filtered. The filtrates were combined, concentrated under reduced pressure and dried to obtain the coarse extract of *Magnolia delavayi.* It was further refined by silica gel column chromatography, eluting with petroleum ether-ethyl acetate gradient to collect fractions rich in parthenolide and costus lactone in stages, which were combined, concentrated, and recrystallized to obtain parthenolide, with a preparation yield of 4.0% and a purity of 96.3%. $^1$H NMR (500 MHz, $CDCl_3$):$\delta$ 6.31 (d, $J$ = 2.9 Hz, 1H), 5.62 (d, $J$ = 3.4 Hz, 1H), 5.20 (d, $J$ = 11.8 Hz, 2H), 3.85 (t, $J$ = 8.6 Hz, 1H), 2.78 (d, $J$ = 8.9 Hz, 1H), 2.45-2.32(m, 2H), 2.22-2.10 (m, 4H), 1.70 (s, 3H), 1.69-1.66 (m, 1H), 1.29 (s, 3H), 1.27 - 1.18 (m, 1H). ESI-MS (m/z): $[M+H]^+$ = 249.1 (calcd: 249.1).

**Preparation of dehydrocostus lactone.** 5 kg of the medicine *Saussurea costus* was crushed into coarse powder, soaked in 8× petroleum ether for 12 h, heated, refluxed and extracted twice with 2 h each time, and filtered. The filtrates were combined, concentrated under reduced pressure and dried to obtain the coarse extract of *Saussurea costus.* It was further refined by silica gel column chromatography, eluting with petroleum ether-ethyl acetate gradient to collect fractions rich in dehydrocostus lactone in stages, which were combined, concentrated, and recrystallized to obtain dehydrocostus lactone, with a preparation yield of 1.0% and a purity of 96.8%. $^1$H NMR (500 MHz):$\delta$ 6.22 (d, $J$ = 3.3 Hz, 1H), 5.49 (d, $J$ = 3.2 Hz, 1H), 5.27 (d, $J$ = 2.0 Hz, 1H), 5.07 (d, $J$ = 2.0 Hz, 1H), 4.90 (s, 1H), 4.82 (s, 1H), 3.97-3.94 (m, 1H), 2.95-2.88 (m, 2H), 2.87 (dd, $J$ = 9.3, 3.0 Hz, 1H), 2.24-2.22 (m, 1H), 2.16-2.13 (m, 1H), 1.99-1.96 (m, 2H), 1.88-1.86 (m, 2H), 1.42-1.40 (m, 2H). ESI-MS (m/z): $[M+H]^+$ = 231.1 (calcd: 231.1).

### Example 2: Synthesis of Compounds 1~9

Although arglabin has a good inhibitory effect on the activation of the NLRP3 inflammasome, the epoxy ring in its structure can be hydrolyzed to open the ring under acidic conditions, and the test found that its degradation ratio reached 50% within 8 h. Therefore, we replaced the epoxy ring in arglabin with cyclopropane, hoping to improve its chemical stability while maintaining its activity.

### Synthesis of Compound 1:

parthenolide

*p*-TSA

$CH_2Cl_2$

**MCL**

$Et_2Zn$, $CH_2I_2$

DME, $CH_2Cl_2$

**1**

Dichloromethane (50 mL), p-toluenesulfonic acid (125 mg, 0.73 mmol) and parthenolide (5 g, 20.16 mmol) were added into a 150 mL round-bottomed flask sequentially, and stirred at room temperature until the completion of the reaction was detected by TLC. The reaction solution was washed with water (10 mL×3) and saturated sodium chloride solution (10 mL×3) sequentially, dried with anhydrous sodium sulfate, and then concentrated under reduced pressure, and purified by silica gel column chromatography to obtain the micheliolide **MCL** with a yield of 90%. $^1H$ NMR (500 MHz, $CDCl_3$):$\delta$ 6.21 (d, $J$ = 3.5 Hz, 1H), 5.51 (d, $J$ = 3.0 Hz, 1H), 3.81 (t, $J$ = 10.5 Hz, 1H), 2.73 (d, $J$ = 10.5 Hz, 1H), 2.68-2.64 (m, 2H), 2.42-2.37 (m, 1H), 2.26-2.16 (m, 3H), 2.11-2.08 (m, 1H), 1.83-1.75 (m, 2H), 1.69 (s, 3H), 1.31 (s, 3H), 1.27-1.25 (m, 1H). ESI-MS (m/z): $[M+Na]^+$ = 271.1 (calcd: 271.1).

Under the condition of an ice bath and nitrogen protection, ethylene glycol dimethyl ether (1.67 mL, 21.26 mmol) was added to anhydrous dichloromethane (67 mL), into which was added 13.3 mL of a diethyl zinc solution (1 M n-hexane solution) after stirring uniformly, and slowly dropped diiodomethane (2.67 mL, 3.11 mmol), and stirred for 10 min to prepare a cyclopropanation reagent. Micheliolide **MCL** (300 mg, 1.21 mmol) and anhydrous dichloromethane (5 mL) were added into another round-bottomed flask, dissolved by stirring, and then protected by nitrogen, and placed on an ice bath. The cyclopropanation reagent above was added to the substrate solution dropwise. After the dropwise addition was completed, the reaction was continued for 1 h, and the reaction was transferred to room temperature for overnight reaction. The reaction solution was quenched with saturated ammonium chloride, filtered, and then washed with water (10 mL×3) and saturated sodium chloride solution (10 mL×3) sequentially, dried with anhydrous sodium sulfate, and then concentrated under reduced pressure, and purified by silica gel column chromatography to obtain Compound **1** with a yield of 75%. $^1H$ NMR (500 MHz, $CDCl_3$):$\delta$6.14 (d, $J$ = 3.5 Hz, 1H), 5.45 (d, $J$ = 3.0 Hz, 1H), 3.82 (t, $J$ = 10.5 Hz, 1H), 2.52-2.48 (m, 1H), 2.26-2.21 (m, 1H), 2.09-1.99 (m, 2H), 1.93-1.89 (m, 1H), 1.88-1.86 (m, 1H, $H_5$), 1.85-1.83 (m, 1H), 1.55 (s, 3H), 1.52-1.44 (m, 2H), 1.26-1.14 (m, 2H), 1.11 (s, 3H), 0.81 (d, $J$ = 4.0 Hz, 1H, $H_{16a}$), 0.54 (d, $J$ = 4.0 Hz, 1H, $H_{16b}$). The ROESY spectrum shows that there is a signal correlation between $H_5$ and $H_{16a}$, which confirms that the cyclopropane is in $\alpha$ configuration. ESI-MS (m/z): $[M+Na]^+$ = 285.2 (calcd: 285.2).

**Preparation of Compound 2:**

**1**

$POCl_3$

pyridine

**2**

Compound **1** (200 mg, 0.81 mmol) and anhydrous pyridine (10 mL) were added into a round-bottomed flask sequentially, protected by nitrogen, and dissolved on an ice water bath, into which was added phosphorus oxychloride (1242 mg, 8.10 mmol) dropwise, and then transferred to room temperature for reaction for 2 h. After the dropwise addition was completed, the reaction was transferred to room temperature for 2 h reaction. The reaction solution was poured into ice water and extracted with ethyl acetate (10 mL×3). The organic layer was washed with a saturated copper sulfate solution (10 mL×6), water (10 mL×3) and saturated sodium chloride solution (10 mL×3) sequentially, dried with anhydrous sodium sulfate, and then concentrated under reduced pressure, and purified by silica gel column chromatography to obtain Compound 2 with a yield of 65%. $^1H$ NMR (500 MHz, $CDCl_3$):$\delta$6.14 (d, $J$ = 3.5 Hz, 1H), 5.59-5.57 (m,1H), 5.44 (d, $J$ = 3.0 Hz, 1H), 3.90 (t, $J$ = 10.5 Hz, 1H), 2.74-2.71 (m, 1H), 2.51-2.46 (m, 1H), 2.32-2.28 (m, 2H), 2.04-2.00 (m, 1H), 1.97 (s, 3H), 1.81-1.77 (m, 1H, $H_5$), 1.54-1.46 (m, 1H), 1.18-1.14 (m, 1H), 1.13 (s, 3H), 0.61 (d, $J$ = 4.0 Hz, 1H, $H_{16a}$), 0.50 (d, $J$ = 4.0 Hz, 1H, $H_{16b}$). The ROESY spectrum shows that there is a signal correlation between $H_5$ and $H_{16a}$, which confirms that the cyclopropane is in $\alpha$ configuration. ESI-MS (m/z): $[M+Na]^+$ = 267.1 (calcd: 267.2).

**Preparation of Compound 3:**

M-chloroperoxybenzoic acid (267.5 mg, 1.55 mmol) and anhydrous dichloromethane (20 mL) were added into a round-bottomed flask sequentially, into which the solution of Compound **2** (248 mg, 1.00 mmol) in dichloromethane (5 mL) was slowly added, and then stirred overnight. The reaction was quenched with a saturated sodium thiosulfate solution, washed with saturated sodium bicarbonate (10 mL×3), water (10 mL×3) and saturated sodium chloride solution (10 mL×3) sequentially, dried with anhydrous sodium sulfate, and then concentrated under reduced pressure, and purified by silica gel column chromatography to obtain Compound **3-1** with a yield of 83%. $^1$H NMR (500 MHz, $CDCl_3$):$\delta$ 6.18 (d, $J$ = 3.5 Hz, 1H), 5.48 (d, $J$ = 3.0 Hz, 1H), 3.79 (t, $J$ = 10.5 Hz, 1H, $H_6$), 2.53-2.48 (m, 1H), 2.28-2.24 (m, 1H, $H_3$), 2.16-2.10 (m, 2H), 2.04-2.01 (m, 1H, $H_5$), 1.73 (s, 3H), 1.56-1.45 (m, 3H), 1.12-1.09 (m, 1H), 1.08 (s, 3H), 0.57 (d, $J$ = 4.0 Hz, 1H, $H_{16a}$), 0.49 (d, $J$ = 4.0 Hz, 1H, $H_{16b}$). The ROESY spectrum shows that there is a signal correlation between $H_5$ and $H_{16a}$, which confirms that the cyclopropane is in $\alpha$ configuration, and the epoxypropane is in $\alpha$ configuration. ESI-MS (m/z): $[M+Na]^+$ = 283.1 (calcd: 283.1).

Intermediate **3-1** (260 mg, 1.00 mmol), methanol (10 mL) and p-toluenesulfonic acid (172 mg, 1.00 mmol) were added to a round-bottomed flask sequentially, and stirred overnight. After concentration, the reaction solution was extracted with ethyl acetate (10 mL×3). The organic layer was washed with saturated sodium bicarbonate (10 mL×3), water (10 mL×3) and saturated sodium chloride solution (10 mL×3) sequentially, dried with anhydrous sodium sulfate, and then concentrated under reduced pressure, and purified by silica gel column chromatography to obtain Compound **3** with a yield of 85%. $^1$H NMR (500 MHz, $CDCl_3$):$\delta$ 6.15 (d, $J$ = 3.5 Hz, 1H), 5.44 (d, $J$ = *3.0* Hz, 1H), 4.25 (t, $J$ = 10.5 Hz, 1H, $H_6$), 3.64-3.62 (m, 1H, $H_3$), 3.41 (s, 3H, $H_{17}$), 2.55-2.48 (m, 1H, $H_7$), 2.41 (s, 1H), 2.22-2.18 (m, 1H), 2.08 (d, $J$ = 11.0 Hz, 1H), 2.06-2.01 (m, 1H), 1.98-1.94 (m, 1H), 1.80-1.75 (m, 1H, $H_5$), 1.61-1.55 (m, 1H), 1.49 (s, 3H), 1.47-1.42 (m, 1H), 1.10 (s, 3H), 0.57 (d, $J$ = 4.0 Hz, 1H, $H_{16a}$), 0.49 (d, $J$ = 4.0 Hz, 1H, $H_{16b}$). ROESY shows that there are signal correlations between $H_3$ and $H_6$, $H_5$ and $H_{16a}$, and $H_7$ and $H_{17}$, which confirms that the cyclopropane is in $\alpha$ configuration, and 3-OH and 4-OMe are in $\alpha$ configuration. ESI-MS (m/z): $[M+Na]^+$ = 315.1 (calcd: 315.1).

**Preparation of Compound 4:**

Tetrahydrofuran (5 mL) and water (1 mL) were added into a round-bottomed flask, into which were added p-toluenesulfonic acid (272 mg, 1.00 mmol) and Intermediate **3-1** (260 mg, 1.00 mmol), and stirred overnight. After concentration, the reaction solution was extracted with ethyl acetate (10 mL×3). The organic layer was washed with saturated sodium bicarbonate (10 mL×3), water (10 mL×3) and saturated sodium chloride solution (10 mL×3) sequentially, dried with anhydrous sodium sulfate, and then concentrated under reduced pressure, and purified by silica gel column chromatography to obtain Compound **4** with a yield of 47%. $^1$H NMR (500 MHz, $CDCl_3$):$\delta$6.16 (d, $J$ = 3.5 Hz, 1H), 5.46 (d, $J$ = 3.0 Hz, 1H), 4.33 (t, $J$ = 10.5 Hz, 1H, $H_6$), 4.12-4.09 (m, 1H, $H_3$), 2.54-2.47 (m, 1H, $H_6$), 2.24-2.19 (m, 1H), 2.10-2.02 (m, 4H), 2.00 (d, $J$ = 5.5 Hz, 1H), 1.87-1.83 (m, 1H, $H_5$), 1.61-1.54 (m, 2H), 1.51 (s, 3H), 1.12 (s, 3H), 0.80 (d, $J$ = 4.0 Hz, 1H, $H_{16a}$), 0.50 (d, $J$ = 4.0 Hz, 1H, $H_{16b}$). The ROESY spectrum shows that there are signal correlations between $H_3$ and $H_6$, $H_6$ and $H_{14}$, and $H_5$ and $H_{16a}$, which confirms that the cyclopropane is in $\alpha$ configuration, and 3-OH and 4-OH are in

$\alpha$ configuration. ESI-MS (m/z): $[M+Na]^+$ =301.2 (calcd: 301.2).

**Preparation of Compound 5:**

Compound **1** (124 mg, 0.50 mmol) and dichloromethane (5 mL) were added into a round-bottomed flask, protected by nitrogen, and dissolved by stirring at -78°C, into which was slowly added DAST reagent (161 mg, 1.00 mmol) dropwise, and continued to be stirred for 10-15 min after the dropwise addition was completed. The reaction solution was quenched by adding water, diluted with dichloromethane, and then washed with water (10 mL×3) and saturated sodium chloride solution (10 mL×3) sequentially, dried with anhydrous sodium sulfate, and then concentrated under reduced pressure, and purified by silica gel column chromatography to obtain Compound **5** with a yield of 55%. $^1$H NMR (500 MHz, $CDCl_3$):$\delta$6.14 (d, $J$ = 3.5 Hz, 1H), 5.43 (d, $J$ = 3.0 Hz, 1H), 4.11 (t, $J$ = 10.5 Hz, 1H), 2.45-2.40 (m, 1H), 2.35-2.31 (m, 1H), 2.23-2.17 (m, 1H), 2.09-2.03 (m, 2H), 2.02-1.97 (m, 1H), 1.85-1.74 (m, 1H, $H_5$), 1.65 (d, $J$ = 21.5 Hz, 3H), 1.54-1.46 (m, 1H), 1.24-1.20 (m, 1H), 1.17 (s, 3H), 1.15-1.12 (m, 1H), 0.80 (d, $J$ = 4.0 Hz, 1H, $H_{16a}$), 0.50 (d, $J$ = 4.0 Hz, 1H, $H_{16b}$). $^{19}$F NMR (470 MHz, $CDCl_3$):$\delta$ -148.17. The ROESY spectrum shows that there is a signal correlation between $H_5$ and $H_{16a}$, which confirms that the cyclopropane is in $\alpha$ configuration. ESI-MS (m/z): $[M+Na]^+$ = 287.2 (calcd: 287.2).

**Preparation of Compound 6:**

Burgess Reagent (282 mg, 1.10 mmol) and anhydrous tetrahydrofuran (10 mL) were added into a round-bottomed flask, protected by nitrogen, and placed on an ice bath. Compound **1** (262 mg, 1.00 mmol) was added. The reaction was stirred for 20 min, and then transferred to room temperature, and continued to be stirred for 3 h. The reaction solution was concentrated, and extracted with ethyl acetate (10 mL×3). The organic layer was washed with water (10 mL×3) and saturated sodium chloride solution (10 mL×3) sequentially, dried with anhydrous sodium sulfate, and then concentrated under reduced pressure, and purified by silica gel column chromatography to obtain Compound **6** with a yield of 75%. $^1$H NMR (500 MHz, $CDCl_3$):$\delta$6.14 (d, $J$ = 3.5 Hz, 1H), 5.60-5.58 (m, 1H), 5.44 (d, $J$ = 3.0 Hz, 1H), 3.90 (t, $J$ = 10.0 Hz, 1H), 2.75-2.71 (m, 1H), 2.52-2.46 (m, 1H), 2.32-2.29 (m, 2H), 2.05-2.00 (m, 1H), 1.98 (s, 3H), 1.81-1.77 (m, 1H), 1.55-1.47 (m, 1H) 1.18-1.14 (m, 1H), 1.13 (s, 3H), 0.63 (d, $J$ = 5.0 Hz, 1H) , 0.51 (d, $J$ = 5.0 Hz, 1H). ESI-MS (m/z): $[M+Na]^+$ = 267.3 (calcd: 267.3).

**Preparation of Compound 7:**

Compound **1** (262 mg, 1.00 mmol) was added into a round-bottomed flask, and protected by nitrogen, into which were added 3 mL of anhydrous dichloromethane and triethylamine (2.7 g, 27.0 mmol) sequentially, and placed on an ice bath, into which was added propionyl chloride dropwise, and warmed to room temperature for overnight reaction. The reaction solution was poured into ice water, and extracted with ethyl acetate (10 mL×3). The organic layer was washed with water (10 mL×3) and saturated sodium chloride solution (10 mL×3) sequentially, dried with anhydrous sodium sulfate, and then concentrated under reduced pressure, and purified by silica gel column chromatography to obtain Compound 7 with a yield of 51%. $^1$H NMR (500 MHz, $CDCl_3$):$\delta$ 6.16 (d, $J$ = 3.5 Hz, 1H), 5.47 (d, $J$ = 3.0 Hz, 1H), 3.82 (t, $J$ = 10.5 Hz, 1H), 2.52-2.48 (m, 1H), 2.27-2.23 (m, 2H), 2.21-2.02 (m, 2H), 1.95-1.92 (m, 1H), 1.90-1.88 (m, 1H), 1.87-1.85 (m, 1H, $H_5$), 1.57 (s, 3H), 1.53-1.49 (m, 2H), 1.24 (t, $J$ = 10.0 Hz, 3H), 1.23-1.14 (m, 2H), 1.11 (s, 3H), 0.59 (d, $J$ = 4.0 Hz, 1H, $H_{16a}$), 0.38 (d, $J$ = 4.0 Hz, 1H, $H_{16b}$). ROESY shows that there is a signal correlation between $H_5$ and $H_{16a}$, which confirms that the cyclopropane is in $\alpha$ configuration. ESI-MS (m/z): $[M+Na]^+$ = 341.2 (calcd: 341.2).

**Preparation of Compound** 8 (not part of the invention):

Under the condition of an ice bath and nitrogen protection, ethylene glycol dimethyl ether (1.67 mL, 21.26 mmol) was added to anhydrous dichloromethane (67 mL), into which was added 13.3 mL of a diethyl zinc solution (1 M n-hexane solution) after stirring uniformly, and slowly dropped diiodomethane (2.67 mL, 3.11 mmol), and stirred for 10 min to prepare a cyclopropanation reagent. Dehydrocostus lactone (300 mg, 1.21 mmol) and anhydrous dichloromethane (5 mL) were added into another round-bottomed flask, protected by nitrogen after dissolving by stirring, and placed on an ice bath. The cyclopropanation reagent above was added to the substrate solution dropwise. After the dropwise addition was completed, the reaction was continued for 1 h, and the reaction was transferred to room temperature for overnight reaction. The reaction solution was quenched with saturated ammonium chloride, filtered, and then washed with water (10 mL×3) and saturated sodium chloride solution (10 mL×3) sequentially, dried with anhydrous sodium sulfate, and then concentrated under reduced pressure, and purified by silica gel column chromatography to obtain Compound **8** with a yield of 65%. $^1$H NMR (500 MHz, $CDCl_3$):$\delta$ 6.24 (d, $J$ = 3.5 Hz, 1H), 5.45 (d, $J$ = 3.0 Hz, 1H), 4.24 (dd, $J$ = 10.8, 8,8 Hz, 1H), 2.79-2.74 (m, 1H), 2.22-2.20 (m, 1H), 2.10-2.06 (m, 1H), 1.95 (dd, $J$ = 10.4, 8,8 Hz, 1H), 1.73-1.67 (m, 2H), 1.63-1.57 (m, 2H), 1.50-1.45 (m, 1H), 1.38-1.35 (m, 2H), 0.98 (s, 1H), 0.64 (s, 1H), 0.50-0.48 (m, 1H), 0.42-0.40 (m, 1H), 0.37-0.27 (m, 4H). ESI-MS (m/z): $[M+Na]^+$ = 259.4 (calcd: 259.4).

**Preparation of Compound 9:**

MCL → $Et_2Zn$, $CH_2I_2$, TFA / $CH_2Cl_2$ → 9

Under the condition of an ice bath and nitrogen protection, 5 mL of a diethyl zinc solution (1 M n-hexane solution) was added into 2.5 mL of dichloromethane. Trifluoroacetic acid (570 mg, 5.00 mmol) was dissolved in 0.8 mL of dichloromethane, which was added dropwise to the above solution, and the reaction was stirred for 20 min. Diiodomethane (1.34 g, 5.00 mmol) was dissolved in 0.8 mL of dichloromethane, which was added dropwise to the above solution, and the reaction was stirred for 20 min. **MCL** (248 mg, 1.00 mmol) was dissolved in 0.8 mL of dichloromethane, which was added to the above solution, and the reaction was stirred and reacted for 5 h. The reaction solution was quenched by adding saturated ammonium chloride, and extracted with ethyl acetate (10 mL×3). The organic layer was washed with water (10 mL×3) and saturated sodium chloride solution (10 mL×3) sequentially, dried with anhydrous sodium sulfate, and then concentrated under reduced pressure, and purified by silica gel column chromatography to obtain Compound **9** with a yield of 12%. $^1H$ NMR (500 MHz, $CDCl_3$): $\delta$ 6.14 (d, $J$ = 3.5 Hz, 1H), 5.44 (d, $J$ = 3.0 Hz, 1H), 3.83 (t, $J$ = 10.5 Hz, 1H), 3.23 (s, 3H), 2.50-2.46 (m, 1H), 2.28-2.23 (m, 1H), 2.02-1.97 (m, 4H), 1.78-1.71 (m, 1H, $H_5$), 1.53 (s, 3H), 1.51-1.39 (m, 2H), 1.13-1.07 (m, 1H), 1.09 (s, 3H), 0.70 (d, $J$ = 4.0 Hz, 1H, $H_{16a}$), 0.46 (d, $J$ = 4.0 Hz, 1H, $H_{16b}$). The ROESY spectrum shows that there is a signal correlation between $H_5$ and $H_{16a}$, which confirms that the cyclopropane is in $\alpha$ configuration. ESI-MS (m/z): $[M+Na]^+$ = 299.2 (calcd: 299.2).

**Example 3: Stability test of Compounds 1~9**

2.00 mg of Compounds **1~9** were precisely weighed. The samples were dissolved in 500 μL of chromatographic methanol, into which were added 1500 μL of artificial gastric juice, mixed well and then dissolved completely by ultrasound. 50 μL of the above solutions were pipetted precisely, diluted by adding 200 μL of chromatographic methanol, filtered and analyzed by HPLC, wherein the HPLC analysis conditions were mobile phase: 65% methanol -35% water, flow rate: 0.8 mL/min, and column temperature: 25°C, and the initial peak areas were recorded. The above solutions were placed on a constant temperature water bath at 37°C, sampled at 8 h, 16 h and 24 h respectively, and analyzed by HPLC. By calculating the peak areas respectively, the stability data of the corresponding samples in artificial gastric juice environment at 8 h, 16 h and 24 h can be obtained.

As shown in Table **1,** the stabilities of Compound **1~9** are significantly improved compared with that of arglabin and dehydrocostus lactone.

**Table 1. Stability of compound in artificial gastric juice environment**

| Group | Non-degradation percentage at 8 h | Non-degradation percentage at 16 h | Non-degradation percentage at 24 h |
|---|---|---|---|
| Compound **1** | 99.08% | 98.76% | 98.16% |
| Compound **2** | 90.92% | 86.62% | 78.26% |
| Compound **3** | 99.98% | 99.87% | 97.77% |
| Compound **4** | 98.15% | 96.84% | 96.21% |
| Compound **5** | 99.91% | 99.85% | 99.84% |
| Compound **6** | 99.89% | 96.80% | 95.72% |
| Compound **7** | 99.31% | 97.85% | 97.65% |
| Compound **8** | 96.33% | 94.29% | 92.86% |
| Compound **9** | 98.36% | 95.58% | 94.08% |
| arglabin | 50.14% | 48.70% | 43.40% |
| dehydrocostus lactone | 68.91% | 65.42% | 57.86% |

**Example 4: Preparation of prodrug (prodrugs include salts)**

**Preparation of Compound 10:**

DMA, $K_2CO_3$ THF

1 10

Compound **1** (262 mg, 1.00 mmol), dichloromethane (30 mL), dimethylamine hydrochloride (815 mg, 10.00 mmol) and potassium carbonate (2764 mg, 20 mmol) were added into a round-bottomed flask, and stirred for 4 h. The reaction solution was filtered, washed with water (10 mL×3) and saturated sodium chloride solution (10 mL×3) sequentially, dried with anhydrous sodium sulfate, and then concentrated under reduced pressure, and purified by silica gel column chromatography (petroleum ether: ethyl acetate: triethylamine = 1: 1: 0.02) to obtain Compound **10** with a yield of 85%. $^1H$ NMR (500 MHz, $CDCl_3$):$\delta$ 3.92 (t, $J$ = 10.5 Hz, 1H), 3.55-3.47 (m, 1H), 3.33-3.29 (m, 1H), 2.38-2.34 (m, 1H), 2.28 (s, 6H), 2.21-2.16 (m, 2H), 1.98 (d, $J$ = 15.0 Hz, 2H), 1.88 (t, $J$ = 5.0 Hz, 2H), 1.72 (d, $J$ = 10.0 Hz, 1H), 1.60-1.52 (m, 2H), 1.50 (s, 3H), 1.20 (t, $J$ = 5.0 Hz, 1H), 1.12 (s, 3H), 1.08-1.04 (m, 1H), 0.76 (d, $J$ = 5.0 Hz, 1H), 0.53 (d, $J$ = 5.0 Hz, 1H). ESI-MS (m/z): $[M+Na]^+$ = 330.2 (calcd: 330.2).

**Preparation of Compound 11:**

DMA, $K_2CO_3$ THF

2 11

The preparation method is the same as that of Compound **10** with Compound **2** and dimethylamine hydrochloride as starting materials, and the yield of Compound **11** is 80%. $^1H$ NMR (500 MHz, $CDCl_3$):$\delta$ 5.52-5.46 (m, 1H), 3.98 (t, $J$ = 10.0 Hz, 1H),3.41-3.32 (m, 1H), 3.20-3.16 (m, 1H), 2.98-2.92 (m, 1H), 2.28 (s, 6H), 2.22-2.19 (m, 1H), 2.10 (d, $J$ = 10.0 Hz, 1H), 2.06-2.02 (m, 1H), 1.98-1.94 (m, 1H), 1.91 (s, 3H), 1.84-1.80 (m, 2H), 1.62-1.56 (m, 1H), 1.19 (s, 3H), 1.14-1.08 (m, 1H), 0.69 (d, $J$ = 5.0 Hz, 1H), 0.51 (d, $J$ = 5.0 Hz, 1H). ESI-MS (m/z): $[M+Na]^+$ = 312.2 (calcd: 312.2).

**Preparation of Compound 12**

DMA, $K_2CO_3$ THF

3 12

The preparation method is the same as that of Compound 10 with Compound **3** and dimethylamine hydrochloride as starting materials, and the yield of Compound **12** is 95%. $^1H$ NMR (500 MHz, $CDCl_3$):$\delta$ 3.96 (t, $J$ = 10.0 Hz, 1H), 3.28-3.23 (m, 1H), 3.15-3.10 (m, 1H), 2.94 (s, 3H), 2.70-2.65 (m, 1H), 2.28 (s, 6H), 2.03-1.99 (m, 1H), 1.91-1.88 (m, 1H), 1.86-1.76 (m, 4H), 1.62-1.56 (m, 1H), 1.48 (s, 3H), 1.28-1.24 (m, 1H), 1.12 (s, 3H), 1.09-1.05 (m, 1H), 0.70 (d, $J$ = 5.0 Hz,

1H), 0.54 (d, $J$ = 5.0 Hz, 1H). ESI-MS (m/z): $[M+Na]^+$ = 360.4 (calcd: 360.5).

**Preparation of Compound 13**

DMA, $K_2CO_3$

THF

**4** **13**

The preparation method is the same as that of Compound **10** with Compound 4 and dimethylamine hydrochloride as starting materials, and the yield of Compound **13** is 90%. $^1$H NMR (500 MHz,$CDCl_3$):$\delta$3.94 (t, $J$ = 10.0 Hz, 1H), 3.38-3.36 (m, 1H), 3.23-3.18 (m, 1H), 3.19-3.15 (m, 2H), 2.78-2.70 (m, 1H), 2.28 (s, 6H), 2.13-2.09 (m, 1H), 1.97-1.92 (m, 1H), 1.90-1.86 (m, 1H), 1.82-1.79 (m, 1H), 1.77-1.75 (m, 1H), 1.62-1.59 (m, 1H), 1.51 (s, 3H), 1.18 (s, 3H), 1.11-1.07 (m, 1H), 0.70 (d, $J$ = 5.0 Hz, 1H), 0.48 (d, $J$ = 5.0 Hz, 1H). ESI-MS (m/z): $[M+Na]^+$ = 368.4 (calcd: 368.4).

**Preparation of Compound 14**

DMA, $K_2CO_3$

THF

**5** **14**

The preparation method is the same as that of Compound **10** with Compound **5** and dimethylamine hydrochloride as starting materials, and the yield of Compound **14** is 83%. $^1$H NMR (500 MHz, $CDCl_3$):$\delta$ 3.96 (t, $J$ = 10.0 Hz, 1H), 3.35-3.30 (m, 2H), 2.80-2.76 (m, 1H), 2.30 (s, 6H), 2.29-2.19 (m, 2H), 2.08 (t, $J$ = 10.0 Hz, 1H), 1.96-1.90 (m, 1H), 1.84-1.82 (m, 2H), 1.81-1.77 (m, 2H), 1.58 (d,J = 21.5 Hz, 3H) 1.46-1.39 (m, 1H), 1.18 (s, 3H), 1.16-1.12 (m, 1H), 0.62 (d, $J$ = 5.0 Hz, 1H), 0.58 (d, $J$ = 5.0 Hz, 1H). ESI-MS (m/z): $[M+Na]^+$ = 332.4 (calcd: 332.4).

**Preparation of Compound 15**

DMA, $K_2CO_3$

THF

**6** **15**

The preparation method is the same as that of Compound **10** with Compound **6** and dimethylamine hydrochloride as starting materials, and the yield of Compound **15** is 91%. $^1$H NMR (500 MHz, $CDCl_3$):$\delta$ 3.99 (t, $J$ = 10.0 Hz, 1H),3.41-3.36 (m, 1H), 3.20-3.18 (m, 1H), 2.82-2.77 (m, 1H), 2.28 (s, 6H), 2.22-2.19 (m, 1H), 2.16-2.10 (m, 2H), 1.98-1.94 (m, 1H), 1.91 (s, 3H), 1.84-1.80 (m, 1H), 1.78-1.75 (m, 1H), 1.62-1.56 (m, 2H), 1.15 (s, 3H), 1.11-1.07 (m, 1H), 0.64 (d, $J$ = 5.0 Hz, 1H), 0.50 (d, $J$ = 5.0 Hz, 1H). ESI-MS (m/z): $[M+Na]^+$ = 312.4 (calcd: 312.4).

**Preparation of Compound 16**

The preparation method is the same as that of Compound **10** with Compound **7** and dimethylamine hydrochloride as starting materials, and the yield of Compound **16** is 80%. $^1$H NMR (500 MHz, $CDCl_3$):$\delta$ 3.92 (t, $J$ = 10.0 Hz, 1H), 3.40-3.30 (m, 2H), 2.83-2.79 (m, 1H), 2.30 (s, 6H), 2.19-2.13 (m, 2H), 2.04-1.97 (m, 2H), 1.95-1.90 (m, 2H), 1.86-1.82 (m, 1H), 1.76-1.73 (m, 1H), 1.60 (s, 3H), 1.56-1.52 (m, 1H), 1.33-1.25 (m, 1H), 1.14 (s, 3H), 1.02 (t, $J$ = 10.0 Hz, 3H), 1.00-0.95 (m, 1H), 0.88-0.82 (m, 1H), 0.48 (d, $J$ = *5.0* Hz, 1H), 0.40 (d, $J$ = 5.0 Hz, 1H). ESI-MS (m/z): $[M+Na]^+$ = 386.5 (calcd: 386.5).

**Preparation of Compound 17** (not part of the invention):

The preparation method is the same as that of Compound **10** with Compound **8** and dimethylamine hydrochloride as starting materials, and the yield of Compound **17** is 78%. $^1$H NMR (500 MHz, $CDCl_3$):$\delta$ 4.15 (dd, $J$ = 9.6, 10.0 Hz, 1H), 2.70 (dd, $J$ = 12.8, 4.8 Hz, 1H), 2.48 (dd, $J$ = 12.8, 4.8 Hz, 1H), 2.36 -2.28 (m, 2H), 2.22 (s, 6H), 2.02-1.98 (m, 2H), 1.84-1.80 (m, 1H), 1.70-1.67 (m, 1H), 1.45-1.30 (m, 5H), 1.11-1.06 (m, 1H), 1.01-0.98 (m, 1H), 0.70-0.68 (m, 1H), 0.46-0.42 (m, 1H), 0.37-0.28 (m, 4H), 0.15-0.11 (m, 2H). ESI-MS (m/z): $[M+Na]^+$ = 327.3 (calcd: 327.3).

**Preparation of Compound 18**

The preparation method is the same as that of Compound **10** with Compound **1** and piperidine as starting materials, and the yield of Compound **18** is 89%. $^1$H NMR (500 MHz, $CDCl_3$):$\delta$ 3.79 (t, $J$ = 10.5 Hz, 1H), 2.79-2.75 (m, 1H), 2.56-2.53 (m, 1H), 2.46-2.40 (m, 3H), 2.39-2.32 (m, 3H), 2.19-2.08 (m, 4H), 1.94-1.85 (m, 3H), 1.77 (d, $J$ = 10.5 Hz, 1H), 1.54 (s, 6H), 1.45-1.39 (m, 4H), 1.12 (s, 3H), 1.09-1.04 (m, 1H), 0.79 (d, $J$ = 4.0 Hz, 1H), 0.52 (d, $J$ = 4.0 Hz, 1H). ESI-MS (m/z): $[M+Na]^+$ = 326.3 (calcd: 326.3).

**Preparation of Compound 19**

The preparation method is the same as that of Compound **10** with Compound **1** and tetrahydropyrrole as starting materials, and the yield of Compound **19** is 87%. $^1$H NMR (500 MHz, $CDCl_3$):$\delta$ 3.79 (t, $J$ = 10.5 Hz, 1H), 2.89-2.80 (m, 2H), 2.57-2.51 (m, 4H), 2.37-2.32 (m, 1H), 2.15-2.06 (m, 3H), 1.94-1.87 (m, 3H), 1.78-1.76 (m, 5H), 1.55 (s, 3H), 1.51-1.39 (m, 3H), 1.11 (s, 3H), 1.09-1.03 (m, 1H), 0.77 (d, $J$ = 4.0 Hz, 1H), 0.51 (d, $J$ = 4.0 Hz, 1H). ESI-MS (m/z): $[M+Na]^+$ =356.3 (calcd: 356.2).

**Preparation of Compound 20**

The preparation method is the same as that of Compound **10** with Compound **1** and tetrahydropyrrole as starting materials, and the yield of Compound **20** is 76%. $^1$H NMR (500 MHz, $CDCl_3$):$\delta$ 3.81 (t, $J$ = 10.5 Hz, 1H), 3.74-3.67 (m, 3H), 2.82-2.79 (m, 1H), 2.65-2.61 (m, 1H), 2.53-2.44 (m, 5H), 2.40-2.35 (m, 1H), 2.21-2.17 (m, 1H), 2.13-2.07 (m, 2H), 1.95-1.86 (m, 3H), 1.78 (d, $J$ = 10.5 Hz, 1H), 1.55 (s, 3H), 1.52-1.39 (m, 2H), 1.31-1.27 (m, 1H), 1.12 (s, 3H), 1.09-1.04 (m, 1H), 0.80 (d, $J$ = 4.0 Hz, 1H), 0.54 (d, $J$ = 4.0 Hz, 1H). ESI-MS (m/z): $[M+Na]^+$ = 372.2 (calcd: 372.2).

**Preparation of Compound 21**

The preparation method is the same as that of Compound **10** with Compound **1** and piperazine as starting materials, and the yield of Compound **21** is 74%. $^1$H NMR (500 MHz, $CDCl_3$):$\delta$ 3.79 (t, $J$ = 10.5 Hz, 1H), 2.82-2.79 (m, 1H), 2.64-2.60 (m, 1H), 2.55-2.42 (m, 5H), 2.38-2.34 (m, 2H), 2.29 (s, 3H), 2.20-2.16 (m, 1H), 2.15-2.05 (m, 2H), 1.93-1.83 (m, 3H), 1.76 (d, $J$ = 10.5 Hz, 1H), 1.54 (s, 3H), 1.49-1.38 (m, 2H), 1.29-1.25 (m, 1H), 1.12 (s, 3H), 1.09-1.03 (m, 1H), 0.78 (d, $J$ = 4.0 Hz, 1H), 0.52 (d, $J$ = 4.0 Hz, 1H). ESI-MS (m/z): $[M+Na]^+$ = 371.2 (calcd: 371.2).

**Preparation of Compound 22**

The preparation method is the same as that of Compound **10** with Compound **1** and piperazine as starting materials, and the yield of Compound **22** is 74%. $^1$H NMR (500 MHz, $CDCl_3$):$\delta$ 3.79 (t, *J* = 10.5 Hz, 1H), 2.83-2.74 (m, 3H), 2.59-2.55 (m, 1H), 2.39-2.35 (m, 2H), 2.20-2.07 (m, 4H), 1.96-1.83 (m, 4H), 1.78 (d, *J* = 10.5 Hz, 1H), 1.55 (s, 3H), 1.51-1.23 (m, 5H), 1.21-1.15 (m, 2H), 1.13 (s, 3H), 1.10-1.05 (m, 1H), 0.93 (d, *J* = 6.5 Hz, 3H), 0.79 (d, *J* = 4.0 Hz, 1H), 0.52 (d, *J* = 4.0 Hz, 1H). ESI-MS (m/z): $[M+Na]^+$ = 384.3 (calcd: 384.3).

**Preparation of Compound 23**

The preparation method is the same as that of Compound **10** with Compound **1** and N-Boc-piperazine as starting materials, and the yield of Compound **23** is 65%. $^1$H NMR (500 MHz, $CDCl_3$):$\delta$ 3.81 (t, *J* = 10.5 Hz, 1H), 2.83-2.79 (m, 1H), 2.66-2.62 (m, 2H), 2.42-2.35 (m, 4H), 2.21-2.16 (m, 1H), 2.13-2.07 (m, 2H), 1.94-1.84 (m, 3H), 1.78 (d, *J = 10.5* Hz, 1H), 1.55 (s, 3H), 1.48 (s, 9H), 1.46-1.44 (m, 1H), 1.40-1.39 (m, 1H), 1.32-1.25 (m, 3H), 1.13 (s, 3H), 1.10-1.04 (m, 2H), 0.92-0.85 (m, 1H), 0.80 (d, *J* = 4.0 Hz, 1H), 0.54 (d, *J* = 4.0 Hz, 1H). ESI-MS (m/z): $[M+Na]^+$ = 471.3 (calcd: 471.3).

**Preparation of hydrochloride 24 of Compound** 10

Compound **10** (307 mg, 1 mmol) was dissolved in dichloromethane (2 mL), and stirred at room temperature for 2 h, into which was then added a hydrochloric acid solution dropwise until the pH value was 4-5, and filtered. The resulting solid was washed with dichloromethane to obtain a white solid, i.e., the hydrochloride (Compound **24)** of Compound **10,** with a yield of 90%. $^1$H NMR (500 MHz, $CD_3OD$):$\delta$ 4.15-4.10 (m, 1H, $H_6$), 3.42-3.37 (m, 1H),3.31-3.26 (m, 1H), 3.04-2.98 (m, 1H, $H_{11}$),2.91 (s, 6H), 2.21-2.12 (m, 2H), 2.02 (d, *J* = 15.0 Hz, 2H), 1.88 (t, *J* = 5.0 Hz, 2H), 1.78 (d, *J = 10.0* Hz, 1H, $H_5$), 1.64-1.54 (m, 2H), 1.52 (s, 3H), 1.26 (t, *J* = 5.0 Hz, 1H), 1.14 (s, 3H), 1.10-1.05 (m, 1H), 0.76 (d, *J* = 5.0 Hz, 1H, $H_{16a}$), 0.53

(d, $J$ = 5.0 Hz, 1H, $H_{16b}$). The ROESY spectrum shows that there are signal correlations between the hydrogens of $H_5$ and $H_{16a}$, as well as the hydrogens of $H_6$ and $H_{11}$, which confirms that the cyclopropane is in $\alpha$ configuration, and 11-H is in $\beta$ configuration. ESI-MS (m/z): $[M+H]^+$ = 344.9(calcd: 344.9).

**Preparation of fumarate 25 of Compound 10**

The fumarate Compound **25** was prepared by using fumaric acid instead of hydrochloric acid according to the preparation method of the hydrochloride of Compound 10. The yield was 80%. $^1$H NMR (500 MHz, $CD_3OD$):$\delta$6.72 (s, 2H), 4.13 (t, $J$ = 10.0 Hz, 1H, $H_6$), 3.41-3.39 (m, 1H), 3.30-3.27 (m, 1H), 3.03-2.98 (m, 1H, $H_{11}$), 2.91 (s, 6H), 2.21-2.12 (m, 2H), 1.94-1.91 (m, 1H), 1.88 (t, $J$ = 5.0 Hz, 2H), 1.84-1.80 (m, 1H), 1.78 (d, $J$ = 15.0 Hz, 1H, $H_5$), 1.64-1.54 (m, 2H), 1.52 (s, 3H), 1.14 (s, 3H), 1.10-1.04 (m, 1H), 0.75 (d, $J$ = 5.0 Hz, 1H, $H_{16a}$), 0.52 (d, $J$ = 5.0 Hz, 1H, $H_{16b}$). The ROESY spectrum shows that there are signal correlations between $H_5$ and $H_{16a}$, as well as the $H_6$ and $H_{11}$, which confirms that the cyclopropane is in $\alpha$ configuration, and 11-H is in $\beta$ configuration. ESI-MS (m/z): $[M+H]^+$ = 424.5 (calcd: 424.5).

**Preparation of hydrochloride 26 of Compound 11**

Using Compound 11 as the starting material, Compound 26 could be prepared according to the preparation method of the hydrochloride of Compound 10, and the yield was 95%. $^1$H NMR (500 MHz, $CD_3OD$):$\delta$ 5.62-5.58 (m, 1H), 4.19 (t, $J$ = 10.0 Hz, 1H, $H_6$), 3.51-3.46 (m, 1H), 3.40-3.36 (m, 1H), 3.12-3.07 (m, 1H, $H_{11}$), 2.98 (s, 6H), 2.82-2.79 (m, 1H), 2.30 (d, $J$ = 10.0 Hz, 1H), 2.26-2.12 (m, 1H), 1.98-1.94 (m, 1H), 1.91 (s, 3H), 1.84-1.82 (m, 2H), 1.80-1.78 (m, 1H, $H_5$), 1.66-1.59 (m, 1H), 1.17 (s, 3H), 1.16-1.10 (m, 1H), 0.61 (d, $J$ = 5.0 Hz, 1H, $H_{16a}$), 0.48 (d, $J$ = 5.0 Hz, 1H, $H_{16b}$). The ROESY spectrum shows that there are signal correlations between $H_5$ and $H_{16a}$, as well as the $H_6$ and $H_{11}$, which confirms that the cyclopropane is in $\alpha$ configuration, and 11-H is in $\beta$ configuration. ESI-MS (m/z): $[M+H]^+$ = 326.9 (calcd: 326.9).

**Preparation of hydrochloride 27 of Compound 12**

Using Compound **12** as the starting material, Compound **27** could be prepared according to the preparation method of the hydrochloride of Compound **10,** and the yield was 95%. $^{1}H$ NMR (500 MHz, $CD_3OD$):$\delta$ 4.39 (t, $J$ = 10.0 Hz, 1H, $H_6$), 3.48-3.43 (m, 1H, $H_7$), 3.40-3.36 (m, 1H), 3.34 (s, 3H, $H_{17}$), 3.10-3.05 (m, 1H, $H_{11}$), 2.98 (s, 6H), 2.23-2.19 (m, 1H, $H_3$), 2.11-2.08 (m, 1H), 1.87-1.80 (m, 3H), 1.79-1.77 (m, 1H, $H_5$),1.65-1.56 (m, 1H), 1.51 (s, 3H), 1.27-1.25 (m, 1H), 1.15 (s, 3H), 1.13-1.08 (m, 1H), 0.74 (d, $J$ = 5.0 Hz, 1H, $H_{16a}$), 0.51 (d, $J$ = 5.0 Hz, 1H, $H_{16b}$). ESI-MS (m/z): $[M+H]^+$ = 374.9 (calcd: 374.9).

**Preparation of hydrochloride 28 of Compound 13**

HCl / $CH_2Cl_2$

**13** → **28**

Using Compound **13** as the starting material, Compound **28** could be prepared according to the preparation method of the hydrochloride of Compound **10,** and the yield was 85%. $^{1}H$ NMR (500 MHz, $CD_3OD$):$\delta$ 4.47 (t, $J$ = 10.0 Hz, 1H, $H_6$), 3.88-3.86 (m, 1H), 3.63-3.58 (m, 1H), 3.49-3.45 (m, 2H), 3.10-3.06 (m, 1H, $H_{11}$), 3.00 (s, 6H), 2.23-2.19 (m, 1H), 1.97-1.92 (m, 1H), 1.90-1.81 (m, 3H), 1.66-1.59 (m, 1H, $H_5$), 1.52 (s, 3H), 1.14 (s, 3H), 1.12-1.10 (m, 1H), 0.73 (d, $J$ = 5.0 Hz, 1H), 0.50 (d, $J$ = 5.0 Hz, 1H). ESI-MS (m/z): $[M+H]^+$ = 360.9 (calcd: 360.9).

**Preparation of hydrochloride 29 of Compound 14**

HCl / $CH_2Cl_2$

**14** → **29**

Using Compound **14** as the starting material, Compound **29** could be prepared according to the preparation method of the hydrochloride of Compound **10,** and the yield was 83%. $^{1}H$ NMR (500 MHz, $CD_3OD$):$\delta$ 4.36 (t, $J$ = 10.0 Hz, 1H, $H_6$), 3.50-3.40 (m, 2H), 3.15-3.11 (m, 1H, $H_{11}$), 3.00 (s, 6H), 2.29-2.19 (m, 2H), 2.11 (t, $J$ = 10.0 Hz, 1H), 2.06-2.00 (m, 1H), 1.94-1.87 (m, 2H), 1.84-1.82 (m, 1H), 1.81-1.78(m,1H, $H_5$) 1.60 (d,J = 21.5 Hz, 3H) 1.60-1.51 (m, 1H), 1.20 (s, 3H), 1.18-1.15 (m, 1H), 0.54 (d, $J$ = 5.0 Hz, 1H, $H_{16a}$), 0.50 (d, $J$ = 5.0 Hz, 1H, $H_{16b}$). ESI-MS (m/z): $[M+H]^+$ = 346.9(calcd: 346.9).

**Preparation of hydrochloride 30 of Compound 15**

HCl / $CH_2Cl_2$

**15** → **30**

Using Compound **15** as the starting material, Compound **30** could be prepared according to the preparation method of the hydrochloride of Compound **10,** and the yield was 80%. $^{1}H$ NMR (500 MHz, $CD_3OD$):$\delta$ 4.19 (t, $J$ = 10.0 Hz,

1H, $H_6$), 3.51-3.46 (m, 1H), 3.40-3.36 (m, 1H), 3.12-3.07 (m, 1H, $H_{11}$), 2.98 (s, 6H), 2.82-2.79 (m, 1H), 2.26-2.12 (m, 2H), 1.98-1.94 (m, 1H), 1.91 (s, 3H), 1.84-1.80 (m, 2H), 1.66-1.59 (m, 2H), 1.17 (s, 3H), 1.16-1.10 (m, 1H), 0.61 (d, $J$ = 5.0 Hz, 1H), 0.48 (d, $J$ = 5.0 Hz, 1H). ESI-MS (m/z): $[M+H]^+$ = 326.9 (calcd: 326.9).

**Preparation of hydrochloride 31 of Compound 16**

**16** → **31**

Using Compound **16** as the starting material, Compound **31** could be prepared according to the preparation method of the hydrochloride of Compound **10,** and the yield was 76%. $^1H$ NMR (500 MHz, $CD_3OD$):$\delta$ 4.22 (t, $J$ = 10.0 Hz, 1H, $H_6$), 3.50-3.38 (m, 2H), 3.13-3.09 (m, 1H, $H_{11}$), 2.99 (s, 6H), 2.69-2.63 (m, 2H), 2.24-2.17 (m, 2H), 1.99-1.93 (m, 2H), 1.86-1.82 (m, 1H, $H_5$), 1.68-1.59 (m, 1H), 1.54-1.45 (m, 1H), 1.36-1.30 (m, 1H), 1.27 (s, 3H), 1.14 (s, 3H), 1.06 (t, $J$ = 10.0 Hz, 3H), 1.04-1.00 (m, 1H), 0.93-0.88 (m, 1H), 0.42 (d, $J$ = 5.0 Hz, 1H, $H_{16a}$), 0.36 (d, $J$ = 5.0 Hz, 1H, $H_{16b}$). ESI-MS (m/z): $[M+H]^+$ = 400.1(calcd: 400.0).

**Preparation of hydrochloride 32 of Compound 17** (not part of the invention):

**17** → **32**

Using Compound **17** as the starting material, Compound **32** could be prepared according to the preparation method of the hydrochloride of Compound **10,** and the yield was 88%. $^1H$ NMR (500 MHz, $CD_3OD$):54.44-4.40 (m, 1H, $H_6$), 3.38-3.33 (m, 1H), 3.26-3.22 (m, 1H), 2.94 -2.90 (m, 1H, $H_{11}$), 2.88 (s, 6H), 2.26-1.98 (m, 2H), 1.96-1.92 (m, 1H), 1.84-1.80 (m, 1H), 1.78-1.75 (m, 1H), 1.58-1.41 (m, 5H), 1.25-1.20 (m, 1H), 1.13-1.08 (m, 1H), 1.01-0.98 (m, 1H), 0.70-0.68 (m, 1H), 0.46-0.42 (m, 1H), 0.33-0.18 (m, 4H), 0.15-0.11 (m, 2H). The ROESY spectrum shows that there is a signal correlation between $H_6$ and $H_{11}$, which confirms that 11-H is in β configuration. ESI-MS (m/z): $[M+H]^+$ = 340.8 (calcd: 340.9).

**Example 5: Compound 25 is converted to Compound 1 in plasma and HEPES Experimental method**

Formulation of HEPES 7.4 solution: 1.6 g of NaCl, 0.074 g of KCl, 0.027 g of $Na_2HPO_4$, 0.2 g of glucose and 1 g of a 4-hydroxyethyl piperazine ethanesulfonic acid (HEPES) solution were placed in 90 mL of distilled water, the pH of which was adjusted to 7.4 with 0.5 M NaOH, and then the volume was adjusted to 100 mL with distilled water.

Preparation of plasma: Mouse plasma was placed in an EP tube filled with heparin sodium in advance, centrifuged at 8000 rpm at 4°C for 10 min, and the supernatant was taken.

Sample analysis: 0.6 mg of Compound **25** was dissolved in 250 μl deionized water. 250 μL of the mouse serum or the HEPES7.4 solution was added into the sample, incubated at 37°C, and sampled at different time points, respectively. 20 μL of samples were taken into EP tubes, into which was added 60 μL of methanol, vortexed to mix well, and centrifuged at 12000 rpm at 4°C for 10 min. The supernatants were taken at 1 h, 2 h, 4 h, 8 h and 12 h, respectively. The samples were analyzed by HPLC with an injection volume of 10 μL, and the corresponding peak areas were recorded. The chromatographic conditions were as follows: the chromatographic column was Hanbang C18 (4.6×250 mm, 5 μm); the mobile

phase was acetonitrile: 10 mmol/mL of ammonium formate solution = 60: 40; the flow rate was 1.0 mL/min; the detection wavelength was 210 nm; and the column temperature was 30°C.

### Experimental result

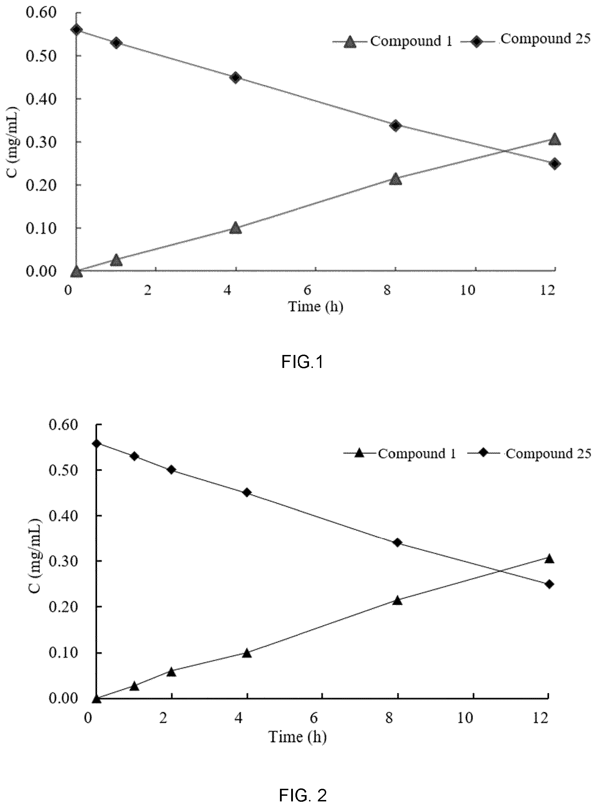

As shown in Fig. 1, at 1 h, 2 h, 4 h, 8 h and 12 h, the contents of Compound **1** in the HEPES buffer solution are gradually increased, and the contents are 5.36%, 11.05%, 19.64%, 39.29% and 55.36%, respectively. The experimental result shows that, Compound **25** can be converted to the prototype Compound **1** as a prodrug in the HEPES buffer solution. As shown in Fig. 2, at 1 h, 2 h, 4 h, 8 h and 12 h, the contents of Compound **1** in the mouse plasma are gradually increased, and the contents are 5.77%, 11.05%, 18.86%, 39.42% and 55.88%, respectively. The experimental result shows that, Compound **25** can be converted to the prototype Compound **1** as a prodrug in the mouse plasma.

Similarly, other prodrug compounds can be converted into corresponding parent drug compounds in plasma and HEPES.

### Example 6: Solubility test of prototype and its prodrug in water

20 μg of Compounds **1~9** and 10 mg of Compounds **24-32** were precisely weighed, respectively, added into 1 mL of deionized water, and completely dissolved by ultrasound. Saturated solutions were formulated, filtered and analyzed by HPLC with a sample volume of 1 μL, 3 μL, 5 μL, 10 μL, 15 μL and 20 μL sequentially, and the standard curves of the corresponding compounds were drawn.

Unsaturated solutions of the above compounds were formulated, dissolved by ultrasound for 4 h, and placed on a water bath at 37 °C to stand for 1 h. The resulting unsaturated solutions were centrifuged. 30 μL of supernatants were removed, diluted by adding 200 μL of deionized water, and then filtered. The samples were analyzed by HPLC, and the solubilities of test compounds **1-9** and **24-32** were obtained by substituting the relevant data into the above measured standard curves.

**Table 2. Water solubility of compound**

| Derivative | Water solubility μg/mL | Salt of 13,13-N,N-dimethyl derivative prodrug | Water solubility mg/mL |
|---|---|---|---|
| Compound **1** | 1140.00 | Compound **24** | 320.70 |
| | | Compound **25** | 310.40 |
| Compound **2** | 0.80 | Compound **26** | 96.60 |
| Compound **3** | 35.48 | Compound **27** | 220.80 |
| Compound **4** | 100.82 | Compound **28** | 248.50 |
| Compound **5** | 25.88 | Compound **29** | 208.40 |
| Compound **6** | 2.58 | Compound **30** | 120.50 |

(continued)

| Derivative | Water solubility μg/mL | Salt of 13,13-N,N-dimethyl derivative prodrug | Water solubility mg/mL |
|---|---|---|---|
| Compound **7** | 17.66 | Compound **31** | 194.60 |
| Compound **8** | 15.30 | Compound **32** | 180.50 |
| Compound **9** | 8.52 | | |
| arglabin | 7.94 | | |
| dehydrocostus lactone | 44.82 | | |

As shown in Table 1, the water solubilities of the prodrug salts **24~32** are at least more than 100 times higher than that of arglabin, dehydrocostunolide and the corresponding parent drugs.

**Example 7: Comparison of Pharmacokinetic Property of Compounds 1 and 25 at Equimolar Dose**

**Experimental material**

**Experimental reagent**

The medicines of the present invention were prepared according to the above Example 1; Tolbutamide (an internal standard, IS), Dalian Meilun Biotechnology Co., Ltd.; Dimethyl sulfoxide, Shanghai Titan Technology Co., Ltd.; Normal saline, Chenxin Pharmaceutical Co., Ltd.; Sodium carboxymethyl cellulose, Aladdin Company; Methanol, Acetonitrile and Formic acid, Merck Company; and Pure water, Hangzhou Wahaha Group Co., Ltd.

**Experimental apparatus**

Freezing centrifuge, Eppendorf Company; Vortex oscillator, Scientific Industries Company; Numerical control ultrasonic cleaner, Kunshan Ultrasonic Instrument Co., Ltd.; Electronic balance, Sartorius Company; Magnetic stirrer, IKA Company; Electronic balance, Changzhou Lucky Electronic Equipment Co., Ltd.; and H-Class/Xevo TQ-S micro LC-MS, Waters Company.

**Experimental animal**

SPF male SD rats, weighing 200 ± 20 g, were provided by Qinglong Mountain Animal Farm, Jiangning District, Nanjing. After purchase, the animals were kept under the condition of ambient temperature of 23~26°C and humidity of 40-60% for 7 days, during which they were free to eat and drink. The animal production license number was SCXK (Zhe) 2019-0002.

**Experimental method**

**Establishment of UPLC-MS/MS determination method**

Chromatographic conditions: Waters Acquity UPLC® BEH $C^{18}$ column (2.1 × 50 mm, 1.7 μm) was used; and gradient elution was performed with 0.1% formic acid aqueous solution as mobile phase A and acetonitrile as mobile phase B (0-1.0 min, 5%-30% B; 1.0-2.0 min, 30%-80% B; 2.0-3.0 min, 80%-80% B; 3.0-4.0 min, 80%-5%B; 4.0-5.0 min, 5%-5% B), total running time: 5 min; flow rate: 0.3 mL/min, column temperature: 30°C, and injection volume: 2 μL.

Mass spectrometry condition: The electrospray ionization source (ESI) was used with a positive ion monitoring mode, the scanning mode was multiple reaction detection mode (MRM), and the ions used for detection were: *m/z* 263.1-227.2 (Compound **1),** *m/z* 308.2 → 116.0 (Compound **24),** and *m/z* 270.9 → 91.0 (IS). The working parameters of MS were set as follows: the capillary voltage was 1000 V, the desolventizing temperature was 600°C, and the desolventizing flow rate was 1000 L/Hr. The cone voltages of Compound **1,** Compound **25** and IS were 26 V, 48 V and 14 V, respectively, and the collision energies were 44 V, 18 V and 30 V, respectively. Masslynx 4.2 was used for data collection and analysis.

**Plasma sample processing**

After investigation by methodology, the plasma samples were pretreated by 1:3 protein precipitation method, wherein methanol was selected as the protein precipitation agent. 50 μL of rat plasma sample was pipetted, into which was added 150 μL of an internal standard methanol solution (0.67 ng/mL), and centrifuged under the condition of 14000 rpm/min and 4°C for 10 min. The supernatant was taken, injected in 2 μL, and analyzed by LC-MS/MS.

**Pharmacokinetic study**

A total of 24 SD rats (male) were randomly divided into intragastric administration groups and tail vein injection groups (Compound **1** and Compound **25),** with 6 rats in each group. Respectively, the rats were administered intragastrically Compound **1** (0.345 mmol/kg) and Compound **25** (0.345 mmol/kg) in equal moles with 0.5% sodium carboxymethyl cellulose (containing 10% DMSO) as solvent; and injected intravenously Compound **1** (0.024 mmol /kg) and Compound **25** (0.024 mmol /kg) in equal moles with normal saline (containing 5% DMSO) as solvent. Blood samples were taken from the orbits of rats after intragastric administration at 0.167, 0.25, 0.5, 0.75, 1, 1.5, 2, 4, 5, 6, 7, 8, 10, 12 and 24 h after administration; and blood samples were taken from the orbits of rats after tail vein injection at 0.033, 0.083, 0.167, 0.25, 0.5, 0.75, 1, 1.5, 2, 4, 6, 8, 10, 12 and 24 hours after administration. The whole blood collected from orbital venous plexus was placed in 1.5 mL EP tube treated with a heparin sodium solution, and centrifuged under the condition of 8000 rpm/min and 4°C for 10 min to obtain plasma, which was stored at -20°C for later use.

**Data analysis**

Pharmacokinetic parameters including elimination half-life ($t_{1/2}$), area under concentration-time curve (*AUC*), mean residence time (MRT), apparent distribution volume ($V_{z/F}$) and plasma clearance rate ($CL_{z/F}$) were calculated by non-compartment model of the DAS (Drugs and Statistics, Version 3.0) software. The maximum concentration ($C_{max}$) and the time to reach the maximum concentration ($T_{max}$) were determined according to the concentration-time curve. All data are expressed as mean ± standard deviation (SD).

**Experimental result**

As shown in Table 3, for intragastric administration of Compound **1** and Compound **25** in equal moles, the AUC value of Compound **1** in blood after intragastric administration of Compound **25** is nearly 2 times that of Compound **1** in blood after intragastric administration of Compound **1,** and the $C_{max}$ value of Compound **1** in blood after intragastric administration of Compound **25** is 10 times that of Compound **1** in blood after intragastric administration of Compound **1,** which shows that the prodrug significantly improves the oral absorption of Compound **1.**

**Table 3. Main pharmacokinetic parameters of Compound 1 in rat blood after intragastric administration of Compound 1** (0.345 mmol/kg) **and Compound 25** (0.345 mmol/kg)

| Parameter | Unit | **1** (Intragastric administration of Compound **1)** | **1** (Intragastric administration of Compound **25)** |
|---|---|---|---|
| $AUC_{(0-t)}$ | ug/L*h | 350.54 ± 134.48 | 666.57 ± 103.39 |
| $AUC_{(0-\infty)}$ | ug/L*h | 351.33 ± 133.66 | 676.70 ± 103.25 |
| $C_{max}$ | ug/L | 42.83 ± 19.73 | 458.37 ± 96.05 |
| $T_{max}$ | h | 8.83 ± 2.04 | 0.38 ± 0.14 |
| $t_{1/2}$ | h | 6.54 ± 0.98 | 2.74 ± 0.90 |
| $MRT_{(0-t)}$ | h | 9.30 ± 0.86 | 1.39 ± 0.26 |
| $MRT_{(0-\infty)}$ | h | 9.37 ± 0.76 | 1.63 ± 0.29 |

**Example 8: Activity test of compound for inhibiting activation of NLRP3 inflammasome**

NLRP3 is an important pattern recognition receptor that can form the NLRP3 inflammasome through the adaptor ASC and pro-caspase-1. After activation, the NLRP3 inflammasome can mediate the activation of caspase-1, thus promoting the maturation and secretion of IL-1β. In order to determine whether the prepared guaianolide sesquiterpene lactone derivatives **1-9** can inhibit the activation of the NLRP3 inflammasome, we used LPS and ATP to induce the

activation of NLRP3 inflammasome, and observed the effects of compounds **1-9** on the IL-1β level caused by the activation of the NLRP3 inflammasome.

**Experimental material**

**Experimental reagent**

The medicines of the present invention were prepared according to the above Example 1; Lipopolysaccharide (LPS) and Adenosine triphosphate (ATP), Sigma Company; Recombinant mouse macrophage colony stimulating factor (rmM-CSF), PeproTech Company; and RPMI 1640 medium, DMEM medium and Fetal bovine serum (FBS).

**Experimental animal**

C57BL/6 mice, female, 6-8 weeks old and weighing 18-20 g, were provided by Qinglong Mountain Animal Farm, Jiangning District, Nanjing, with the production license number of SCXK (Su) 2017-0001.

**Experimental method**

**Isolation and culture of mouse bone marrow-derived macrophages (BMDMs)**

C57BL/6 mice were killed by cervical dislocation, and then soaked in 75% alcohol for 5-10 min. Subsequently, two hind legs of the mice were cut off with scissors. Next, the meat was removed, and the leg bones were left, which were then washed with PBS for three times. After severing the bone at both ends, a sterile syringe filled with cold serum-free RPMI 1640 medium was used to flush the bone marrow into a 15 mL centrifuge tube. Subsequently, the bone marrow was centrifuged at 1500 rpm for 5 min, and the supernatant was discarded. The residue was resuspended with 1 mL of erythrocyte lysate, blown repeatedly, and then stood for 7 min to lyse erythrocytes. After centrifugation at 1500 rpm for 5 min, the supernatant was discarded. The residue was resuspended with the RPMI 1640 medium containing 100 ng/ml of rMM-CSF, and then transferred to a 6-well culture plate for culture. After 6-7 days, the state of the cells could be observed to be a long spindle shape, indicating that they are in good condition and can be used for subsequent experiments.

**Establishment of activation model of NLRP3 inflammasome**

The BMDMs were seeded on 6-well cell culture plates and then treated with 100 ng/mL ultrapure LPS for 3 h. The old medium was replaced by fresh serum-free medium and treated with arglabin (1, 3, 10, 30, 60, and 120 nM) for 1 h. Subsequently, the cells were stimulated with 5 mM ATP for 45 min. The supernatants were collected into 1.5 mL EP tubes for subsequent detection of IL-1β level.

**Table 4. Inhibitory effect of derivatives 1~9 and their prodrugs on activation of NLRP3 inflammasome**

| Derivative | $IC_{50}$ (nM) | Prodrug | $IC_{50}$ (nM) |
|---|---|---|---|
| **1** | +++ | **24** | ++ |
| | | **25** | ++ |
| **2** | +++ | **26** | ++ |
| **3** | +++ | **27** | ++ |
| **4** | +++ | **28** | ++ |
| **5** | +++ | **29** | ++ |
| **6** | +++ | **30** | + |
| **7** | +++ | **31** | ++ |
| **8** | +++ | **32** | ++ |
| **9** | +++ | | |
| arglabin | +++ | | |
| Note: + stands for 80 nM <$IC_{50}$<100 nM, ++ stands for 40 nM< $IC_{50}$<80 nM, and +++ stands for $IC_{50}$<40 nM | | | |

In BMDMs, LPS and ATP were used to induce the activation of NLRP3 inflammasome, and the effects of Compounds **1~9** and **24~32** on the protein level of IL-1β were investigated. As shown in Table 4, derivatives **1~9** all have better inhibitory activities on IL-1β, which are close to the positive compound arglabin; and the activities of prodrugs are decreased slightly.

**Example 9: Anti-ulcerative colitis activity test of Compound 1 and its dimethyl fumarate prodrug 25**

**Experimental reagent**

The medicines of the present invention were prepared according to the above Example 1; Dextran sulfate sodium (DSS), MP Biomedicals Company; Mesalazine sustained-release granules (5-aminosalicylic acid, 5-ASA), Ethypharm Pharmaceutical Company, France; Sodium carboxymethyl cellulose (CMC-Na), Xilong Chemical Plant, Shantou City, Guangdong Province; Myeloperoxidase (MPO) kit, Nanjing Institute of Bioengineering; O-toluidine, Shanghai Jingchun Biochemical Technology Co., Ltd.; and Hydrogen peroxide ($H_2O_2$) and Glacial acetic acid, Nanjing Chemical Reagent Co., Ltd.

**Experimental animal**

C57BL/6 mice, female, 6-8 weeks old and weighing 18-20 g, were provided by Qinglong Mountain Animal Farm, Jiangning District, Nanjing, with the production license number of SCXK (Su) 2017-0001. The animals were free to eat and drink, and fed with standard pellet feed at room temperature of 22 ± 2°C and humidity of 45 ± 10%. After accommodation for 3 days, they were used for experiments.

**Experimental method**

**Establishment of mouse colitis model and administration according** to **groups**

The mice were randomly divided into 7 groups, one of which was randomly selected as the normal group, and the other 6 groups were the model group, arglabin (20 nmol/kg), Compound **1** (20 and 40 nmol/kg) and Compound **25** (20 and 40 nmol/kg) groups, respectively, with 6 mice in each group. Except for the normal group, the other mice were free to drink 2.5% DSS for 7 days, which was then replaced to distilled water to be drunk freely for 3 days to establish the UC model. From the first day of the model establishment, arglabin (20 nmol/kg/d), Compound **1** (20 and 40 nmol/kg) and Compound **25** (20 and 40nmol/kg) were administered intragastrically for 10 consecutive days. The mice in the normal group and model group were administered intragastrically the same volume of the solvent 0.5% CMC-Na.

**Score of disease activity index**

The general living conditions of the mice in each group were observed and the disease activity indexs (DAI) were evaluated. The daily specific observation indicator was weight, stool property and occult blood state of mice, and then the scores of the weight loss, stool property and occult blood state were added to determine the average value, so as to obtain the DAI score of each mouse, that is, DAI = (weight loss score + stool property score + occult blood state score) /3, to evaluate the disease activity.

As shown in Table 5, the scoring criteria are as follows:

**Table 5. Score of disease activity index**

| Score | Weight loss (%) | Stool property | Bleeding in stool |
|---|---|---|---|
| 0 | none | normal | negative |
| 1 | 1-5 | - | - |
| 2 | 6-10 | soft | positive |
| 3 | 11-15 | - | - |
| 4 | >15 | diarrhea | bleeding with naked eyes |

**Test method of occult blood:** Using o-toluidine method, a little feces was picked up into a 24-well culture plate with a cotton swab, into which was first added 200 μL of the solution of o-toluidine glacial acetic acid, and then quickly added 200 μL of 3% hydrogen peroxide solution. Those turned blue-brown within 2 minutes were positive.

**Specimen collection**

1 h after the last administration, blood was collected from the venous plexus of fundus oculi, stood at room temperature for 2 h, and centrifuged at 3000 rpm for 20 min. The serum was aspirated, aliquoted, and frozen at -70°C for subsequent use. After the blood collection, the mice were killed by cervical dislocation, and the abdominal cavity was opened. The colon tissues were removed 1 cm away from the anus, rinsed twice with pre-cooled PBS, and frozen at -70°C for subsequent use.

**Determination of colon length**

The shortening of colon length is one of the main characteristics of DSS-induced UC model mice. After dissecting the abdominal cavities of the UC mice, the colon and distal ileum tissues were dissociated. The external morphological changes of the colon tissues were observed, the colon length was measured and recorded, and the photos were taken.

**Determination of MPO activity**

40 mg of colon tissues of the mice in each group was taken, into which was added 400 μL of pre-cooled normal saline to prepare tissue homogenates. After centrifugation at 4°C and 1200 rpm for 5 min, the supernatants were collected. According to the method described in the kit instructions, various reagents were added sequentially, and finally the absorbance value of each well was determined at the wavelength of 460 nm of the microplate reader to calculate the MPO activity.

MPO activity (U/g wet weight) = (OD value of test tube-OD value of control tube) /11.3 × sampling amount (g)

**Statistics**

All data were expressed by Means ± S.E.M., and the significances of the differences were analyzed by ANOVA. Those with significant differences by ANOVA were further compared for differences between groups by one-way ANOVA and Dunnett's test. P value less than 0.05 was considered to have significant difference.

**Experimental result**

The mice in the Normal group had normal activities and defecation, and their weights were increased slowly; and the mice given DSS showed symptoms such as loose stools and semi-formed stools without sticking to anuses over the days of the experiment, with reduced activities and significant body weight loss. The disease activity indexes were evaluated by observing the body weight losses, stool properties and hematochezia of the mice every day. As shown in Table 6, compared with the DSS group, Compound **25** (40 nmol/kg) significantly reduced the increased DAI score of colitis caused by DSS, and the activity of the prodrug **25** was significantly stronger than that of the original drug **1** at the same dosage.

**Effect on DAI score**

**Table 6. DAI score**

| | | Group | | | | | |
|---|---|---|---|---|---|---|---|
| Days | Normal | DSS | **1** (20 nmol/kg) | **1** (40 nmol/kg) | **25** (20 nmol/kg) | **25** (40 nmol/kg) | Argla bin (20 nmol/ kg) |
| 1 | 0.00±0.00 | 0.00±0.00 | 0.00±0.00 | 0.00±0.00 | 0.00±0.00 | 0.00±0.00 | 0.00±0.00 |
| 2 | 0.00±0. 00 | 0.50±0. 17 | 0.33±0.1 4 | 0.33±0.1 9 | 0.27±0.09 | 0.16±0.10 | 0.36% 0.20 |
| 3 | 0.00±0. 00 | 0.83±0. 3t!!i | 0.75±0.2 8 | 0.58±0.2 1 | 0.63±0.14 | 0.54±0.11 | 0.65± 0.20 |
| 4 | 0.00±0. 00 | 1.50±0. 35## | 1.08±0.3 7 | 1.00±0.1 9 | 0.96±0.16 | 1.20±0.14 * | 1.17± 0.19 |
| 5 | 0.00±0. 00 | 2.08±0. 50## | 1.75±0.5 0 | 1.25±0.3 4 | 1.02±0.23 * | 0.61±0.23 * | 1.39± 0.13* |
| 6 | 0.00±0. 00 | 2.75±0. 39## | 2.25±0.4 4 | 1.67±0.3 6* | 1.56±0.41 * | 1.07±0.25 ** | 1.62± 0.10** |

(continued)

| | | Group | | | | | |
|---|---|---|---|---|---|---|---|
| Days | Normal | DSS | **1** (20 nmol/kg) | **1** (40 nmol/kg) | **25** (20 nmol/kg) | **25** (40 nmol/kg) | Argla bin (20 nmol/ kg) |
| 7 | 0.00±0. 00 | 3.08±0. 16## | 2.50±0.1 7 | 1.83±0.4 4* | 2.28±0.23 * | 1.38±0.25 ** | 2.07± 0.17** |
| 8 | 0.00±0. 00 | 3.17±0. 32## | 2.75±0.3 9 | 1.92±0.5 0* | 2.46±0.26 ** | 1.57±0.28 ** | 2.32± 0.10** |
| 9 | 0.00±0. 00 | 2.17±0. 17## | 1.83±0.1 7 | 1.17±0.3 2* | 1.31±0.18 * | 1.05±0.25 ** | 1.45± 0.13** |
| 10 | 0.00±0. 00 | 1.41±0. 21 | 1.25±0.1 6 | 0.50±0.2 2* | 0.80±0.16 ** | 0.35±0.16 ** | 0.92± 0.06** |
| Note: Compared with the Normal group, ##*P*< 0.01; and compared with the DSS group, **P*< 0.05, and ***P< 0.01.* | | | | | | | |

**Effect on colon length**

**Table 7. Colon length**

| Group | Number | Length (cm) |
|---|---|---|
| Normal | 6 | 7.35 ± 0.06 |
| DSS | 6 | 5.38 ± 0.35## |
| **1** (20 nmol/kg/d) | 6 | 6.15 ± 0.35 |
| **1** (40 nmol/kg/d) | 6 | 6.78 ± 0.28** |
| **25**(20 nmol/kg/d) | 6 | 6.51±0.25** |
| **25** (40 nmol/kg/d) | 6 | 7.26±0.26** |
| arglabin (20 nmol/kg/d) | 6 | 6.50±0.15** |
| Note: Compared with the Normal group, ##*P*< 0.01; and compared with the DSS group, ***P*< 0.01. | | |

As shown in Table 7, compared with the mice in the Normal group, the colon length of the mice in the DSS group was significantly shorter. Intragastric administration of Compound **25** (40 nmol/kg) significantly inhibited the shortening of colon length, and the activity of the prodrug **25** was significantly stronger than that of the original drug **1** at the same dosage.

**Effect on MPO activity in colon tissue**

The granulocytes can synthesize myeloperoxidase MPO in bone marrow before entering the blood circulation, and the latter is stored in azurophil granules. MPO can kill pathogenic microorganisms and regulate inflammatory responses by producing hypochlorous acid. MPO accounts for about 5% of dry weight of cells, and this feature can be used to determine the number of neutrophils in tissues. As shown in Table 8, compared with the mice in the Normal group, the MPO activities of the mice in the DSS group are significantly increased. Intragastric administration of **25** (40 nmol/kg) significantly reduced the MPO activities in colon tissues of mice, and the activity of prodrug **25** was significantly stronger than that of the original drug **1** at the same dosage.

**Table 8. MPO activity in colon tissue**

| Group | Number | MPO activity (U/g tissue) |
|---|---|---|
| Normal | 6 | 0.62±0.05 |
| DSS | 6 | 1.72±0.28## |
| **1** (20 nmol/kg) | 6 | 1.40±0.31 |
| **1** (40 nmol/kg) | 6 | 1.08 ± 0.12** |
| **25** (20 nmol/kg) | 6 | 1.29 ± 0.11** |
| **25**(40 nmol/kg) | 6 | 0.99 ± 0.18** |

(continued)

| Group | Number | MPO activity (U/g tissue) |
|---|---|---|
| arglabin (20 nmol/kg) | 6 | 0.95±0.18** |
| Note: Compared with the Normal group, ##$P$< 0.01; and compared with the DSS group, **$P$< 0.01. | | |

**Example 10: Anti-acute gouty arthritis activity test of Compound 25**

**Experimental reagent**

The medicines of the present invention were prepared according to the above Example 2; Colchicine tablets, Xishuangbanna Banna Pharmaceutical Co., Ltd.; Prednisolone acetate tablets, Tianjin Xinyi Jinjin Pharmaceutical Co., Ltd.; Sodium carboxymethyl cellulose (CMC-Na), Sinopharm Chemical Reagent Co.Ltd.; Pentobarbital sodium, (China National Pharmaceutical Group) Shanghai Chemical Reagent Company; Sodium urate (MSU), Sigma-Aldrich; and IL-1β radioimmunoassay kit and IL-18 radioimmunoassay kit, Beijing Huaying Institute of Biotechnology.

**Experimental animal**

SPF SD rats, female, weighing 200-220 g, were provided by Hangzhou Medical College, with the production license number of SCXK (Zhe) 2019-0002. Animal use license number: SYXK (Su) 2019-0004. The animals were free to eat and drink, and fed with standard pellet feed at room temperature of 22 ± 2°C and humidity of 45 ± 10%. After accommodation for 3 days, they were used for experiments.

**Experimental method**

**Rat acute gouty arthritis model**

During the adaptive feeding period, 2% pentobarbital sodium was used to anesthetize the rats by intraperitoneal injection at a dose of 0.25 mL/100 g. The circumferences of the ankle joints of the original left hind limbs of the rats were measured. After the adaptive feeding, 2% pentobarbital sodium was used for intraperitoneal injection anesthesia at a dose of 0.25 mL/100 g. After the anesthesia, 8 rats were randomly selected as the blank group, and normal saline was injected into the joint cavities of their left hind limbs. The other rats were injected with 200 μL sodium urate into the joint cavities of the left hind limbs . 6 h after the model establishment, the rats were anesthetized with pentobarbital sodium, and the joint circumference was measured with a 2 mm wide paper strip. According to the joint swelling ratio, the rats were divided into seven groups. A blank group (equal volume of CMC-Na); a model and positive control group (equal volume of CMC-Na), a colchicine group (0.5 mg /kg), a prednisolone group (3.125 mg/kg) and a low, medium and high dose group (1.5 mg/kg, 5.0 mg/kg and 15 mg/kg) of Compound **25,** with 8 rats in the blank group and 10 rats in each of the other groups.

**Administration according to groups**

Administration was carried out once at 10.5 h and 22.5 h after the model establishment, respectively. Rats in the blank group and the model group were administered intragastrically CMC-Na (0.5%), rats in the colchicine group were administered intragastrically a colchicine solution, rats in the prednisolone group were administered intragastrically a prednisolone solution, and rats in each dose group of Compound **25** were administered intragastrically Compound **25** with different concentrations, and the administration volume was 10 mL/kg.

**Evaluation of joint swelling degree**

Joint swelling is one of the main characteristics of acute gouty arthritis model rats induced by MSU. Before the model establishment, and at 6 h, 12 h and 24 h after the model establishment, the circumferences of the ankle joints of the left hind limbs were measured respectively, and the joint swelling ratios were calculated. The circumferences at 0.5 mm below the ankle joints of the left hind feet of rats in each group were measured with a 2 mm wide paper strip and a ruler, the measurement was repeated twice, and the average value was taken to calculate the joint swelling ratio.

Joint swelling ratio = (joint circumference at test time point - initial joint circumference)/initial joint circumference

### Statistics

All data were expressed by Means ± S.E.M., and the significances of the differences were analyzed by ANOVA. Those with significant differences by ANOVA were further compared for differences between groups by one-way ANOVA and Dunnett's test. *P* value less than 0.05 was considered to have significant difference.

### Experimental result

**Table 9. Effect of Compound 1 on joint swelling ratio in rats (Mean ± SEM)**

| Group | Number | 6 h | 12 h | 24 h |
|---|---|---|---|---|
| blank group | 8 | 5.31 ±0.87 | 2.01±0.25 | 2.15±0.41 |
| model group | 10 | 16.41±1.36## | 33.09±2.83## | 31.43±2.37##. |
| colchicine group | 10 | 16.15±1.37 | 29.10±3.17 | 23.28±2.14* |
| prednisolone group | 10 | 15.78±1.22 | 25.43±2.03 | 18.48±1.66** |
| low dose group of Compound **25** | 10 | 15.90±0.91 | 30.89±1.70 | 23.55±2.23* |
| medium dose group of Compound **25** | 10 | 15.94±1.27 | 29.69±2.92 | 21.45±2.01** |
| high dose group of Compound **25** | 10 | 16.00±1.33 | 28.84±3.401 | 19.93±2.59** |
| Note: Compared with the blank group, ##*P*< 0.01, and compared with the model group, **P*< 0.05, and ***P< 0.01.* | | | | |

### Effect on joint swelling ratio induced by MSU

As shown in Table 9, the swelling ratios of ankle joints of rats in the model group at 6 h, 12 h and 24 h after the model establishment are significantly higher than that in the control group; and the swelling ratios of ankle joints of rats in each administration group at 1.5 h after the first administration (12 h after the model establishment) are all decreased, but there is no significant difference. The swelling ratios of ankle joints of rats in each administration group at 1.5 h after the second administration (24 h after the model establishment) are all decreased significantly, suggesting that Compound **25** can significantly reduce the swelling ratios of ankle joints of rats with acute gouty arthritis induced by MSU.

## Claims

1. Guaianolide sesquiterpene lactone derivatives of general formula I, or a pharmaceutically acceptable salt thereof, wherein

I

$R_1$ and $R_2$ together form a double bond; or $R_1$ is hydrogen or deuterium, $R_2$ is

$$-CH_2N(R_3)(R_4),$$

wherein $R_3$ and $R_4$ are alkyl groups respectively, or $R_3$, $R_4$ and a N atom form a 3~9 membered ring structure on which there are groups selected from an alkyl and ester group;

$R_5$ is methyl; and $R_6$ is a hydroxyl, alkoxy, ester group, halogen or forms a double bond with an adjacent carbon atom;

$R_7$ is hydrogen or hydroxyl;

$R_8$ is methyl; and $R_9$ is connected with $R_{10}$ to form cyclopropane.

**2.** The guaianolide sesquiterpene lactone derivatives or a pharmaceutically acceptable salt thereof according to claim 1, which is selected from the following compounds:

1 2 3 4

5 6 7 9

10 11 12 13

14 15 16

R= 18 19 20 21 22 23

**3.** The guaianolide sesquiterpene lactone derivatives or a pharmaceutically acceptable salt thereof according to claim 1, wherein the pharmaceutically acceptable salt is selected from a hydrochloride, a sulfate, a phosphate, a maleate, a fumarate and a citrate.

**4.** The guaianolide sesquiterpene lactone derivatives or a pharmaceutically acceptable salt thereof according to claim 3,

wherein the pharmaceutically acceptable salt is selected from a hydrochloride and a fumarate:

24 25 26 27

28 , 29 , 30 , and 31 .

5. A pharmaceutical composition comprising the guaianolide sesquiterpene lactone derivatives or a pharmaceutically acceptable salt thereof according to any one of claims 1-4, and a pharmaceutically acceptable carrier.

6. The guaianolide sesquiterpene lactone derivatives or a pharmaceutically acceptable salt thereof according to any one of claims 1-4 and the pharmaceutical composition according to claim 5 for use in treating NLRP3 inflammasome-associated diseases.

7. The guaianolide sesquiterpene lactone derivatives or a pharmaceutically acceptable salt thereof according to any one of claim 1-4 and the pharmaceutical composition according to claim 5 for use in treating NLRP3 inflammasome-associated diseases, wherein the NLRP3 inflammasome-associated diseases are selected from immune diseases, autoimmune diseases, malignant tumor, skin diseases, cardiovascular diseases, liver-associated diseases, kidney system-associated diseases, gastrointestinal tract-associated diseases, central nervous system diseases, metabolic diseases, endocrine-associated diseases, respiratory diseases, lymphatic system diseases, inflammation, infectious diseases, ocular diseases, psychological disorders and pain.

8. The guaianolide sesquiterpene lactone derivatives or a pharmaceutically acceptable salt thereof according to any one of claim 1-4 and the pharmaceutical composition according to claim 5 for use in treating NLRP3 inflammasome-associated diseases, wherein the NLRP3 inflammasome-associated diseases are selected from: (1) Cryopyrin-associated periodic syndrome CAPS: a Muckle-Wells syndrome MWS, a familial cold autoinflammatory syndrome FCAS, and a neonatal-onset multisystem inflammatory disease NOMID; (2) autoinflammatory diseases: familial Mediterranean fever FMF, a TNF receptor-associated periodic syndrome TRAPS, mevalonate kinase deficiency MKD, a hyperimmunoglobulin D and periodic fever syndrome HIDS, deficiency of interleukin-1 receptor DIRA, a Majeed syndrome, pyogenic arthritis, pyoderma gangrenosum and acne PAPA, haploinsufficiency of A20 HA20, pediatric granulomatous arthritis PGA, PLCG2-associated antibody deficiency and immune dysregulation PLAID, PLCG2-associated autoinflammation, antibody deficiency and immune dysregulation APLAID, and sideroblastic anaemia with B-cell immunodeficiency, periodic fever and developmental delay SIFD; (3) Sweet's syndrome: chronic nonbacterial osteomyelitis CNO, chronic recurrent multifocal osteomyelitis CRMO, and a synovitis, acne, pustulosis, hyperostosis, and osteitis syndrome SAPHO; (4) autoimmune diseases: multiple sclerosis MS, type 1 diabetes, psoriasis rheumatoid arthritis, a Behcet's disease, a Sjogren's syndrome, and a Schnitzler syndrome; (5) respiratory system diseases: a chronic obstructive pulmonary disease COPD, steroid-resistant asthma, asbestosis, silicosis, and cystic fibrosis; (6) central nervous system diseases: a Parkinson's disease, an Alzheimer's disease, a motor neuron disease, a Huntington's disease, cerebral malaria, and brain injury from pneumococcal meningitis; (7) metabolic diseases: type 2 diabetes, atherosclerosis, obesity, gout, and pseudogout; (8) ocular diseases: an ocular epithelium disease, age-related macular degeneration AMD, corneal infection, uveitis, and xerophthalmia; (9) kidney-associated diseases: a chronic kidney disease, oxalate nephropathy, and diabetic nephropathy; (10) liver-associated diseases: non-alcoholic steatohepatitis, and an alcoholic liver disease; (11) skin-associated inflammatory reaction: contact hypersensitivity, and sunburn; (12) joint-associated inflammatory reaction: osteoarthrosis, systemic juvenile idiopathic arthritis, an adult Still's disease, and relapsing polychondritis; (13) viral infection: Dengue virus and Zika virus, influenza, and HIV virus; (14) hidradenitis suppurativa (HS), and other cyst-causing skin diseases; (15) cancer: lung

cancer, pancreatic cancer, gastric cancer, a myelodysplastic syndrome, abdominal aortic aneurysm, and leukemia; and (16) polymyositis, colitis, pericarditis, worm infection, bacterial infection, wound healing, depression, stroke, myocardial infarction, hypertension, a Dressler's syndrome, and ischemia-reperfusion injury.

**9.** The guaianolide sesquiterpene lactone derivatives or a pharmaceutically acceptable salt thereof according to any one of claim 1-4 and the pharmaceutical composition according to claim 5 for use in treating NLRP3 inflammasome-associated diseases according to claim 8, wherein the colitis is ulcerative colitis.

**10.** The guaianolide sesquiterpene lactone derivatives or a pharmaceutically acceptable salt thereof according to any one of claim 1-4 and the pharmaceutical composition according to claim 5 for use in treating NLRP3 inflammasome-associated diseases according to claim 6, wherein the NLRP3 inflammasome-associated disease is acute gouty arthritis.

## Patentansprüche

**1.** Guaianolid-Sesquiterpenlactonderivate der allgemeinen Formel I oder ein pharmazeutisch akzeptables Salz davon, wobei

I

$R_1$ und $R_2$ zusammen eine Doppelbindung bilden; oder $R_1$ Wasserstoff oder Deuterium ist, $R_2$

ist, wobei $R_3$ und $R_4$ jeweils Alkylgruppen sind oder $R_3$, $R_4$ und ein N-Atom eine 3-9-gliedrige Ringstruktur bilden, an der Gruppen vorhanden sind, die aus einer Alkyl- und Estergruppe ausgewählt sind;
$R_5$ Methyl ist; und $R_6$ ein Hydroxyl, ein Alkoxy, eine Estergruppe, ein Halogen ist oder eine Doppelbindung mit einem benachbarten Kohlenstoffatom bildet;
$R_7$ Wasserstoff oder Hydroxyl ist;
$R_8$ Methyl ist; und $R_9$ mit $R_{10}$ verbunden ist, um Cyclopropan zu bilden.

**2.** Guaianolid-Sesquiterpenlactonderivate oder ein pharmazeutisch akzeptables Salz davon gemäß Anspruch 1, das aus den folgenden Verbindungen ausgewählt ist:

5 6 7 9

10 11 12 13

14 15 16

R= 18 19 20 21 22 23

3. Guaianolid-Sesquiterpenlactonderivate oder ein pharmazeutisch akzeptables Salz davon gemäß Anspruch 1, wobei das pharmazeutisch akzeptable Salz aus einem Hydrochlorid, einem Sulfat, einem Phosphat, einem Maleat, einem Fumarat und einem Citrat ausgewählt ist.

4. Guaianolid-Sesquiterpenlactonderivate oder ein pharmazeutisch akzeptables Salz davon gemäß Anspruch 3, wobei das pharmazeutisch akzeptable Salz aus einem Hydrochlorid und einem Fumarat ausgewählt ist:

24
25
26
27

**28** , **29** , **30** und **31** .

**5.** Eine pharmazeutische Zusammensetzung, die die Guaianolid-Sesquiterpenlactonderivate oder ein pharmazeutisch akzeptables Salz davon gemäß einem der Ansprüche 1-4 und einen pharmazeutisch akzeptablen Träger beinhaltet.

**6.** Guaianolid-Sesquiterpenlactonderivate oder ein pharmazeutisch akzeptables Salz davon gemäß einem der Ansprüche 1-4 und pharmazeutische Zusammensetzung gemäß Anspruch 5 zur Verwendung bei der Behandlung von mit NLRP3-Inflammasom assoziierten Erkrankungen.

**7.** Guaianolid-Sesquiterpenlactonderivate oder ein pharmazeutisch akzeptables Salz davon gemäß einem der Ansprüche 1-4 und pharmazeutische Zusammensetzung gemäß Anspruch 5 zur Verwendung bei der Behandlung von mit NLRP3-Inflammasom assoziierten Erkrankungen, wobei die mit NLRP3-Inflammasom assoziierten Erkrankungen aus Immunerkrankungen, Autoimmunerkrankungen, malignem Tumor, Hauterkrankungen, Herz-Kreislauf-Erkrankungen, Leber-assoziierten Erkrankungen, Nierensystem-assoziierten Erkrankungen, Magen-Darm-Trakt-assoziierten Erkrankungen, Erkrankungen des zentralen Nervensystems, Stoffwechselerkrankungen, endokrin-assoziierten Erkrankungen, Atemwegserkrankungen, Lymphsystemerkrankungen, Entzündung, Infektionserkrankungen, Augenerkrankungen, psychologischen Störungen und Schmerzen ausgewählt sind.

**8.** Guaianolid-Sesquiterpenlactonderivate oder ein pharmazeutisch akzeptables Salz davon gemäß einem der Ansprüche 1-4 und pharmazeutische Zusammensetzung gemäß Anspruch 5 zur Verwendung bei der Behandlung von mit NLRP3-Inflammasom assoziierten Erkrankungen, wobei die mit NLRP3-Inflammasom assoziierten Erkrankungen aus Folgendem ausgewählt sind: (1) Cryopyrin-assoziiertem periodischem Syndrom CAPS: einem Muckle-Wells-Syndrom MWS, einem familiären autoinflammatorischen Kältesyndrom FCAS und einer neonatal beginnenden entzündlichen Multisystemerkrankung NOMID; (2) autoinflammatorischen Erkrankungen: familiärem Mittelmeerfieber FMF, einem TNF-Rezeptor-assoziierten periodischen Syndrom TRAPS, Mevalonatkinase-Mangel MKD, einem Hyper-IgD-Syndrom mit periodischem Fieber HIDS, Mangel an Interleukin-1-Rezeptor DIRA, einem Majeed-Syndrom, pyogener Arthritis, Pyoderma gangraenosum und Akne PAPA, Haploinsuffizienz von A20 HA20, pädiatrischer granulomatöser Arthritis PGA, PLCG2-assoziiertem Antikörpermangel und Immundysregulation PLAID, PLCG2-assoziierter Autoinflammation, Antikörpermangel und Immundysregulation APLAID und sideroblastischer Anämie mit B-Zell-Immundefizienz, periodischem Fieber und Entwicklungsverzögerung SIFD; (3) Sweet-Syndrom: chronischer nichtbakterieller Osteomyelitis CNO, chronischer rezidivierender multifokaler Osteomyelitis CRMO und einem Syndrom der Synovitis, Akne, Pustulose, Hyperostose und Osteitis SAPHO; (4) Autoimmunerkrankungen: Multipler Sklerose MS, Typ-1-Diabetes, Psoriasis, rheumatoider Arthritis, einer Behçet-Krankheit, einem Sjögren-Syndrom und einem Schnitzler-Syndrom; (5) Erkrankungen des Atemwegsystems: einer chronisch obstruktiven Lungenerkrankung COPD, steroidresistentem Asthma, Asbestose, Silikose und zystischer Fibrose; (6) Erkrankungen des zentralen Nervensystems: einer Parkinson-Krankheit, einer Alzheimer-Krankheit, einer Motoneuronerkrankung, einer Huntington-Krankheit, zerebraler Malaria und Hirnschädigung durch Pneumokokken-Meningitis; (7) Stoffwechselerkrankungen: Typ-2-Diabetes, Atherosklerose, Adipositas, Gicht und Pseudogicht; (8) Augenerkrankungen: einer Augenepithelerkrankung, altersbedingter Makuladegeneration AMD, Hornhautinfektion, Uveitis und Xerophthalmie; (9) Nieren-assoziierten Erkrankungen: einer chronischen Nierenerkrankung, Oxalat-Nephropathie und diabetischer Nephropathie; (10) Leber-assoziierten Erkrankungen: nichtalkoholischer Steatohepatitis und einer alkoholischen Lebererkrankung; (11) Haut-assoziierter Entzündungsreaktion:
Kontaktüberempfindlichkeit und Sonnenbrand; (12) Gelenk-assoziierter Entzündungsreaktion: Osteoarthrose, systemischer juveniler idiopathischer Arthritis, einem Morbus Still des Erwachsenen und rezidivierender Polychondritis; (13) Virusinfektion: Dengue-Virus und Zika-Virus, Influenza und HIV-Virus; (14) Hidradenitis suppurativa (HS) und anderen zystenverursachenden Hauterkrankungen; (15) Krebs: Lungenkrebs, Bauchspeicheldrüsenkrebs, Magenkrebs, einem myelodysplastischen Syndrom, abdominalem Aortenaneurysma und Leukämie; und (16) Polymyositis, Kolitis, Perikarditis, Wurminfektion, bakterieller Infektion, Wundheilung, Depression, Schlaganfall, Myokardinfarkt, Hypertonie, einem Dressler-Syndrom und Ischämie-Reperfusionsschaden.

**9.** Guaianolid-Sesquiterpenlactonderivate oder ein pharmazeutisch akzeptables Salz davon gemäß einem der An-

sprüche 1-4 und pharmazeutische Zusammensetzung gemäß Anspruch 5 zur Verwendung bei der Behandlung von mit NLRP3-Inflammasom assoziierten Erkrankungen gemäß Anspruch 8, wobei die Kolitis Colitis ulcerosa ist.

**10.** Guaianolid-Sesquiterpenlactonderivate oder ein pharmazeutisch akzeptables Salz davon gemäß einem der Ansprüche 1-4 und pharmazeutische Zusammensetzung gemäß Anspruch 5 zur Verwendung bei der Behandlung von mit NLRP3-Inflammasom assoziierten Erkrankungen gemäß Anspruch 6, wobei die mit NLRP3-Inflammasom assoziierte Erkrankung akute Gichtarthritis ist.

## Revendications

**1.** Des dérivés de lactone de sesquiterpène de guaianolide de formule générale I, ou un sel pharmaceutiquement acceptable de ceux-ci, où

I

$R_1$ et $R_2$ forment ensemble une double liaison ; ou $R_1$ est l'hydrogène ou le deutérium, $R_2$ est

où $R_3$ et $R_4$ sont des groupes alkyle respectivement, ou $R_3$, $R_4$ et un atome N forment une structure cyclique à 3 à 9 chaînons sur laquelle il y a des groupes sélectionnés dans un groupe alkyle et ester ;
$R_5$ est le méthyle ; et $R_6$ est un groupe hydroxyle, alcoxy, ester, un halogène ou forme une double liaison avec un atome de carbone adjacent ;
$R_7$ est l'hydrogène ou l'hydroxyle ;
$R_8$ est le méthyle ; et $R_9$ est relié à $R_{10}$ pour former un cyclopropane.

**2.** Les dérivés de lactone de sesquiterpène de guaianolide ou un sel pharmaceutiquement acceptable de ceux-ci selon la revendication 1, qui est sélectionné parmi les composés suivants :

1
2
3
4
5
6
7
9

10 11 12 13

14 15 16

R= 18 19 20 21 22 23

3. Les dérivés de lactone de sesquiterpène de guaianolide ou un sel pharmaceutiquement acceptable de ceux-ci selon la revendication 1, où le sel pharmaceutiquement acceptable est sélectionné parmi un chlorhydrate, un sulfate, un phosphate, un maléate, un fumarate et un citrate.

4. Les dérivés de lactone de sesquiterpène de guaianolide ou un sel pharmaceutiquement acceptable de ceux-ci selon la revendication 3, où le sel pharmaceutiquement acceptable est sélectionné parmi un chlorhydrate et un fumarate :

24 25 26 27

28 , 29 , 30 et 31 .

5. Une composition pharmaceutique comprenant les dérivés de lactone de sesquiterpène de guaianolide ou un sel pharmaceutiquement acceptable de ceux-ci selon l'une quelconque des revendications 1 à 4, et un support pharmaceutiquement acceptable.

6. Les dérivés de lactone de sesquiterpène de guaianolide ou un sel pharmaceutiquement acceptable de ceux-ci selon l'une quelconque des revendications 1 à 4 et la composition pharmaceutique selon la revendication 5 pour une utilisation dans le traitement de maladies associées à l'inflammasome NLRP3.

7. Les dérivés de lactone de sesquiterpène de guaianolide ou un sel pharmaceutiquement acceptable de ceux-ci selon l'une quelconque des revendications 1 à 4 et la composition pharmaceutique selon la revendication 5 pour une utilisation dans le traitement de maladies associées à l'inflammasome NLRP3, où les maladies associées à l'inflammasome NLRP3 sont sélectionnées parmi des maladies immunitaires, des maladies auto-immunes, une tumeur maligne, des maladies de la peau, des maladies cardiovasculaires, des maladies associées au foie, des maladies associées au système rénal, des maladies associées au tractus gastro-intestinal, des maladies du système nerveux central, des maladies métaboliques, des maladies à association endocrinienne, des maladies respiratoires, des maladies du système lymphatique, une inflammation, des maladies infectieuses, des maladies oculaires, des troubles psychologiques et une douleur.

8. Les dérivés de lactone de sesquiterpène de guaianolide ou un sel pharmaceutiquement acceptable de ceux-ci selon l'une quelconque des revendications 1 à 4 et la composition pharmaceutique selon la revendication 5 pour une utilisation dans le traitement de maladies associées à l'inflammasome NLRP3, où les maladies associées à l'inflammasome NLRP3 sont sélectionnées parmi : (1) le syndrome périodique associé à la cryopyrine CAPS : un syndrome de Muckle et Wells MWS, un syndrome auto-inflammatoire du froid familial FCAS, et une maladie inflammatoire multisystémique néonatale NOMID ; (2) des maladies auto-inflammatoires : la fièvre familiale méditerranéenne FMF, un syndrome périodique associé au récepteur de TNF TRAPS, une déficience en mévalonate kinase MKD, un syndrome d'hyperimmunoglobuline D et de fièvre périodique HIDS, une déficience en récepteur d'interleukine-1 DIRA, un syndrome de Majeed, l'arthrite pyogénique, le pyoderma gangrenosum et l'acné PAPA, l'haplo-insuffisance en A20 HA20, l'arthrite granulomateuse pédiatrique PGA, une déficience en anticorps et un dérèglement immunitaire associés à PLCG2 PLAID, une auto-inflammation associée à PLCG2, une déficience en anticorps et un dérèglement immunitaire APLAID, et une anémie sidéroblastique avec immunodéficience en cellules B, une fièvre périodique et un retard de développement SIFD ; (3) le syndrome de Sweet : l'ostéomyélite non bactérienne chronique CNO, l'ostéomyélite multifocale récurrente chronique CRMO, et un syndrome de la synovite, de l'acné, de la pustulose, de l'hyperostose, et de l'ostéite SAPHO ; (4) des maladies auto-immunes : la sclérose en plaques MS, le diabète de type 1, le psoriasis, la polyarthrite rhumatoïde, une maladie de Behcet, un syndrome de Sjogren, et un syndrome de Schnitzler ; (5) des maladies du système respiratoire : une maladie pulmonaire obstructive chronique COPD, l'asthme résistant aux stéroïdes, l'asbestose, la silicose, et la fibrose kystique ; (6) des maladies du système nerveux central : une maladie de Parkinson, une maladie d'Alzheimer, une maladie des neurones moteurs, une maladie de Huntington, le paludisme cérébral, et une lésion cérébrale due à une méningite à pneumocoques ; (7) des maladies métaboliques : le diabète de type 2, l'athérosclérose, l'obésité, la goutte, et la pseudogoutte ; (8) des maladies oculaires :
une maladie de l'épithélium oculaire, la dégénérescence maculaire liée à l'âge AMD, une infection cornéenne, l'uvéite, et la xérophtalmie ; (9) des maladies associées au rein : une maladie rénale chronique, une néphropathie oxalate, et une néphropathie diabétique ; (10) des maladies associées au foie : la stéatohépatite non alcoolique, et une maladie alcoolique du foie ; (11) une réaction inflammatoire associée à la peau :
une hypersensibilité de contact, et un coup de soleil ; (12) une réaction inflammatoire associée aux articulations : l'ostéoarthrose, l'arthrite idiopathique juvénile systémique, une maladie de Still de l'adulte, et une polychondrite récidivante ; (13) une infection virale : le virus de la dengue et le virus Zika, la grippe, et le virus VIH ; (14) l'hidradénite suppurée (HS), et d'autres maladies cutanées provoquant des kystes ; (15) un cancer :
le cancer du poumon, le cancer du pancréas, le cancer de l'estomac, un syndrome myélodysplasique, un anévrisme aortique abdominal, et la leucémie ; et (16) une polymyosite, une colite, une péricardite, une infection au ver, une infection bactérienne, une cicatrisation de plaies, une dépression, un accident vasculaire cérébral, un infarctus du myocarde, une hypertension, un syndrome de Dressler, et une lésion d'ischémie-reperfusion.

9. Les dérivés de lactone de sesquiterpène de guaianolide ou un sel pharmaceutiquement acceptable de ceux-ci selon l'une quelconque des revendications 1 à 4 et la composition pharmaceutique selon la revendication 5 pour une utilisation dans le traitement de maladies associées à l'inflammasome NLRP3 selon la revendication 8, où la colite est une colite ulcéreuse.

10. Les dérivés de lactone de sesquiterpène de guaianolide ou un sel pharmaceutiquement acceptable de ceux-ci selon l'une quelconque des revendications 1 à 4 et la composition pharmaceutique selon la revendication 5 pour une utilisation dans le traitement de maladies associées à l'inflammasome NLRP3 selon la revendication 6, où la maladie associée à l'inflammasome NLRP3 est l'arthrite goutteuse aiguë.

0.60
0.50
0.40
0.30
0.20
0.10
0.00
C (mg/mL)
0
2
4
6
8
10
12
Time (h)
Compound 1
Compound 25


FIG.1

0.60
0.50
0.40
0.30
0.20
0.10
0.00
C (mg/mL)
0
2
4
6
8
10
12
Time (h)
Compound 1
Compound 25


FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Front Immunol*, 2019, vol. 10, 2538
- *Cell*, 2016, vol. 165, 792-800
- *Nat Rev Drug Discov*, 2018, vol. 17, 588-606
- *Immunol Rev*, 2015, vol. 265, 35-52
- *Int J Mol Sci*, 2019, vol. 20, 3328
- *Front Immunol*, 2019, vol. 10, 276
- *Nat Med*, 2015, vol. 21, 248-255
- *J Clin Invest*, 2020, vol. 130, 1961-1976
- *Cell Metab*, 2020, vol. 31, 580-591
- *Circ Res*, 2018, vol. 122, 1722-1740
- *J Hepatol*, 2017, vol. 66, 1037-1046
- *Ageing Res Rev*, 2020, vol. 64, 101192
- *Autophagy*, 2019, vol. 15, 1860-1881
- *Mucosal Immunol*, 2019, vol. 12, 1150-1163
- *J Clin Invest*, 2018, vol. 128, 1793-1806
- *Immunology*, 2020, vol. 160, 78-89
- *J Inflamm (Lond)*, 2015, vol. 12, 41
- *Nat Commun*, 2020, vol. 11, 4243
- *Front Immunol*, 2020, vol. 11, 570251
- *BiochemBiophys Res Commun*, 2016, vol. 477, 329-335
- *Pharmaceutics*, 2020, vol. 12, 867
- *Arthritis Rheumatol*, 2020, vol. 72, 1192-1202
- *Food Chem Toxicol*, 2020, vol. 144, 111588
- *EMBO Rep*, 2020, vol. 21, e49666
- *Int Immunopharmacol*, 2020, vol. 81, 106257
- *Cells*, 2019, vol. 8, 1389
- *Cell Prolif*, 2021, vol. 54, e12973
- *Circulation*, 2015, vol. 131, 1061-1070
- *J Pharmacol Exp Ther*, 2016, vol. 357, 487-494
- *J Nat Prop*, 1999, vol. 62, 1068-1071